# FASCICULE DE BREVET EUROPEEN

(11) **EP 0 698 105 B1**
(45) Date de publication et mention de la délivrance du brevet: **15.03.2006**
(21) Numéro de dépôt: 94915589.9
(22) Date de dépôt: 05.05.1994
(51) Int. Cl.: C12N 15/75, C12N 15/32, C12N 15/90, C12N 1/21, C12N 15/56, C12R 1/07, C12R 1/25

(54) **SEQUENCES DE NUCLEOTIDES POUR LE CONTROLE DE L'EXPRESSION DE SEQUENCES D'ADN DANS UN HOTE CELLULAIRE**
NUKLEOTID-SEQUENZEN ZUR KONTROLLE DER EXPRIMIERUNG VON DNS-SEQUENZEN IN EINEM ZELLULÄREM WIRT
NUCLEOTIDE SEQUENCES FOR THE CONTROL OF THE EXPRESSION OF DNA SEQUENCES IN A CELLULAR HOST

(30) Priorité: 05.05.1993 FR 9305387
(43) Date de publication de la demande: 28.02.1996
(73) Titulaire: INSTITUT PASTEUR, 75724 Paris Cédex 15 (FR); INSTITUT NATIONAL DE LA RECHERCHE AGRONOMIQUE (INRA), 75341 Paris Cédéx 07 (FR)
(72) Inventeur: LERECLUS, Didier, F-75015 Paris (FR); AGAISSE, Hervé, F-75015 Paris (FR)
(74) Mandataire: Gutmann, Ernest
(86) Numéro de dépôt international: PCT/FR1994/000525
(87) Numéro de publication internationale: WO 1994/025612

(56) Documents cités:
- EP-A- 0 318 143
- MOLECULAR MICROBIOLOGY, vol.4, no.12, 1990 pages 2087 - 2094 DANCOCSIK, C. ET AL. 'Activation of a cryptic crystal protein gene of Bacillus thuringiensis subspecies kurstaki by gene fusion and determination of the crystal protein insecticidal specificity'
- MOLECULAR AND GENERAL GENETICS, vol.214, 1988, BERLIN DE pages 365 - 372 DONOVAN, W. ET AL. 'Isolation and characterization of EG2158, a new strain of Bacillus thuringiensis toxic to coleopteran larvae, and nucleotide sequence of the toxin gene' cité dans la demande
- JOURNAL OF BACTERIOLOGY, vol.175, no.10, 10 Mai 1993, WASHINGTON D.C., US pages 2952 - 2960 TEIXEIRA DE SOUZA, M. ET AL. 'Full expression of the cryIIIA toxin gene of Bacillus thuringiensis requires a distant upstream DNA sequence affecting transcription'
- MOLECULAR MICROBIOLOGY 13 (1). 1994. 97-107 AGAISSE, H. & LERECLUS, D. 'Structural and functional analysis of the promoter region involved in full expression of the cryIIIA toxin gene of bacillus thuringiensis.'

## Description

L'invention a pour objet des séquences de nucléotides de bactéries, particulièrement de bactéries Gram⁺ telles des bactéries de type Bacillus et plus particulièrement des séquences de nucléotides du gène cryIIIA pour le contrôle de l' expression de séquences d'ADN dans un hôte cellulaire.

Le gène cryIIIA code pour une toxine spécifique des coléoptères et est faiblement exprimé dans Bacillus thuringiensis lorsqu'il est cloné dans un plasmide à faible nombre de copies.

Bacillus thuringiensis est une bactérie Gram-positive qui produit des quantités significatives de protéines sous forme de cristal ayant une activité toxique vis-à-vis des larves d'insectes. On connaît deux groupes de protéines de cristal, établis selon les séquences d'acides aminés et les spécificités de toxicité :
1) la classe des toxines Cry (I, II, III, etc...) qui ont des similitudes de structures ;
2) la classe des toxines Cyt, qui n'est pas apparentée à la classe cry (Höfte, H., et al 1989. Microbiol. Rev. 53:242-255).

Ces toxines de B. thuringiensis sont intéressantes de façon générale en vue de l'élaboration de bio-pesticides, et également dans la mesure où la synthèse des protéines du cristal est connue pour être parfaitement coordonnée avec la phase de sporulation de l'organisme, rendant cet organisme intéressant pour l'étude de la régulation génétique chez les bactéries sporulantes Gram-positives.

Différents mécanismes impliqués dans la régulation de la synthèse des protéines du cristal de B. thuringiensis ont été décrits. Le haut niveau d'expression de ces protéines est attribué, au moins en partie, à la stabilité de l'ARNm. Certains auteurs ont attribué la stabilité de cet ARNm à la présence en aval du gène de la toxine, d'une structure jouant un rôle de terminateur qui pourrait agir comme un rétro-régulateur positif en protégeant l'extrémité 3' de l'ARNm de la dégradation par les nucléases, augmentant ainsi la demi-vie des transcrits (Wong, H.C. et al 1986. Proc. Natl. Acad. Sci. USA 83:3233-3237).

On a également émis l'hypothèse de la présence de polypeptides impliqués dans la synthèse de protéines du cristal, polypeptides qui agiraient soit en dirigeant le repliement de la protéine sous forme d'une protéine ayant une conformation stable ou pour protéger ces protéines de la dégradation protéolytique.

Des études au microscope électronique et des études biochimiques de la sporulation de B. thuringiensis montrent que la production de la protéine de cristal est dépendante de la sporulation et se situe dans le compartiment de la cellule mère (Ribier, J. et al 1973.Ann. Inst.Pasteur 124A:311-344).

Récemment, 2 facteurs sigma, sigma 35 et sigma 28, qui dirigent spécifiquement la transcription des gènes cryIA, ont été isolés et caractérisés. Ces séquences d'acides aminés montrent une identité de 88 et 85 % avec les facteurs sigma E et sigma K de Bacillus subtilis, respectivement (Adams, L. F., 1991. J. Bactériol. 173:3846-3854). Ces facteurs sigma sont produits exclusivement dans les cellules sporulantes et sont capables de fonctionner dans le compartiment de la cellule mère, confirmant que l'expression des gènes de la protéine du cristal est contrôlée dans le temps et dans l'espace. Ainsi dans l'art antérieur, on a conclu que l'expression du gène au cours du temps est, au moins en partie, assurée par l'activation successive des facteurs sigma spécifiques de la sporulation. Jusqu'à présent trois groupes de promoteurs ont été identifiés. Deux de ces groupes comprennent des promoteurs reconnus par des facteurs sigma déterminés, et selon l'art antérieur, les facteurs sigma liés au troisième groupe de promoteurs (dont celui du gène cryIIIA) n'ont pas été identifiés (Lereclus, D., et al 1989. American Society for Microbiology, Washington, D.C.).

Enfin le nombre de copies du plasmide portant le gène semble être un facteur important pour l'expression du gène cry dans B. thuringiensis. Dans la souche sauvage B. thuringiensis, les gènes cry sont localisés sur des plasmides de grande taille, présents à un faible nombre de copies.

La demande de brevet européen EP 0318143 décrit la présence, dans B. thuringiensis var. tenebrionis, d'un fragment BamHI de 5,9 kb portant le gène de la δ-endotoxine.

Des expériences de clonage d'un fragment de 3 kb HindIII, cloné dans un plasmide à faible nombre de copies, conduisent à une production faible de toxines dans une souche acristalifère (cry⁻) de B. thuringiensis. Au contraire, de grandes quantités de toxines sont synthétisées lorsque le gène est cloné dans des plasmides à haut nombre de copies (Arantes, O. et al 1991. Gene 108:115-119).

### Résumé de l'invention

L'invention a pour objet des moyens permettant d'obtenir un haut niveau d'expression de la protéine codée par le gène cryIIIA et plus généralement des moyens permettant de contrôler le niveau d'expression de séquences d'ADN codant pour une protéine d'intérêt déterminé, dans des souches bactériennes, préférablement des souches Gram⁺, telles des souches de Bacillus, cette expression pouvant être obtenue lorsque la séquence d'ADN codante est située sur un vecteur en particulier sur un plasmide à faible nombre de copies.

De façon générale, l'invention concerne un système d'expression comprenant une séquence d'ADN, susceptible d'intervenir dans le contrôle de l'expression d'une séquence codante de nucléotides, et obtenue en associant deux séquences de nucléotides distinctes et intervenant de façons différentes mais, de façon préférable, non dissociables, dans le contrôle de l'expression de la séquence codante. La première séquence de nucléotides présente une activité promotrice, alors que la deuxième séquence, initiée par l'activité promotrice de la première, intervient pour augmenter l'expression du gène. La séquence d'ADN de l'invention permet d'atteindre un haut niveau d'expression de la partie codante d'un gène dans une bactérie, particulièrement une bactérie de type Gram⁺.

La première séquence de nucléotides du système d'expression de la présente invention, identifiée dans le cadre de la présente demande comme étant le promoteur, comprend soit le promoteur de la souche hôte dans laquelle le gène d'intérêt à exprimer est introduit, soit un promoteur exogène, fonctionnel dans l'hôte utilisé. La deuxième séquence de nucléotides du sytème d'expression de l'invention, identifiée dans la présente demande comme étant la "région aval", désigne toute séquence, préférablement située entre le promoteur et la séquence codante d'un gène à exprimer, susceptible de jouer un rôle, particulièrement au niveau post-transcriptionnel, lors de l'expression du gène. Plus particulièrement, la région aval n'agit pas directement sur la traduction de la séquence codante à exprimer.

De façon préférée, la "région aval" comprend une séquence nucléotidique, particulièrement une séquence S2 ou analogue à S2, comportant une région essentiellement complémentaire à l'extrémité 3' de l'ARN, particulièrement l'ARN 16S, des ribosomes de bactéries, particulièrement de bactéries Gram⁺ de type Bacillus.

Les nucléotides composant la séquence d'ADN selon l'invention peuvent être consécutifs ou non sur l'enchaînement à partir duquel est définie la séquence d'ADN.

Dans le cadre de la présente demande, l'expression "séquence d'ADN susceptible d'intervenir dans le contrôle de l'expression d'une séquence codante de nucléotides" traduit la capacité de cette séquence d'ADN, d'initier ou d'empêcher l'expression de la séquence codante ou de réguler cette expression notamment au niveau de la quantité du produit exprimé.

Une séquence d'ADN selon l'invention est telle que la séquence codante de nucléotides qu'elle contrôle est placée immédiatement en aval, en phase de lecture avec elle ou est au contraire séparée de cette séquence d'ADN par un fragment de nucléotides.

L'invention concerne donc une séquence d'ADN de contrôle de l'expression d'une séquence codante d'un gène dans une cellule hôte, la séquence d'ADN est caractérisée en ce qu'elle comprend un promoteur, et une séquence nucléotidique ou région aval particulièrement située en aval dudit promoteur et en amont de ladite séquence codante. La séquence nucléotidique ou région aval comporte une région essentiellement complémentaire à l'extrémité 3' d'un ARN ribosomal bactérien. La séquence d'ADN de l'invention est susceptible d'intervenir pour augmenter l'expression de la séquence codante, placée en aval, dans un hôte cellulaire.

Les inventeurs ont identifié une séquence d'ADN du type décrit précédemment, susceptible d'intervenir dans le contrôle de l'expression de la séquence codante du gène cryIIIA, et permettant notamment d'obtenir un haut niveau de l'expression lorsque la séquence codante est placée sur un plasmide à faible nombre de copies.

L'invention concerne donc également une séquence d'ADN caractérisée par les propriétés suivantes :
- elle est comprise dans un enchaînement d'ADN d'une longueur de 1692 pb environ, délimité par les sites de restriction HindIII-Pst1 (fragment H₂-P₁), tel qu'obtenu par digestion partielle du fragment BamHI de 6 kb porté par le gène cryIIIA de Bacillus thuringiensis souche LM79 ;
- elle est susceptible d'intervenir dans le contrôle de l'expression d'une séquence codante de nucléotides placée en aval, dans un hôte cellulaire, particulièrement un hôte cellulaire bactérien du type Bacillus thuringiensis et/ou Bacillus subtilis.

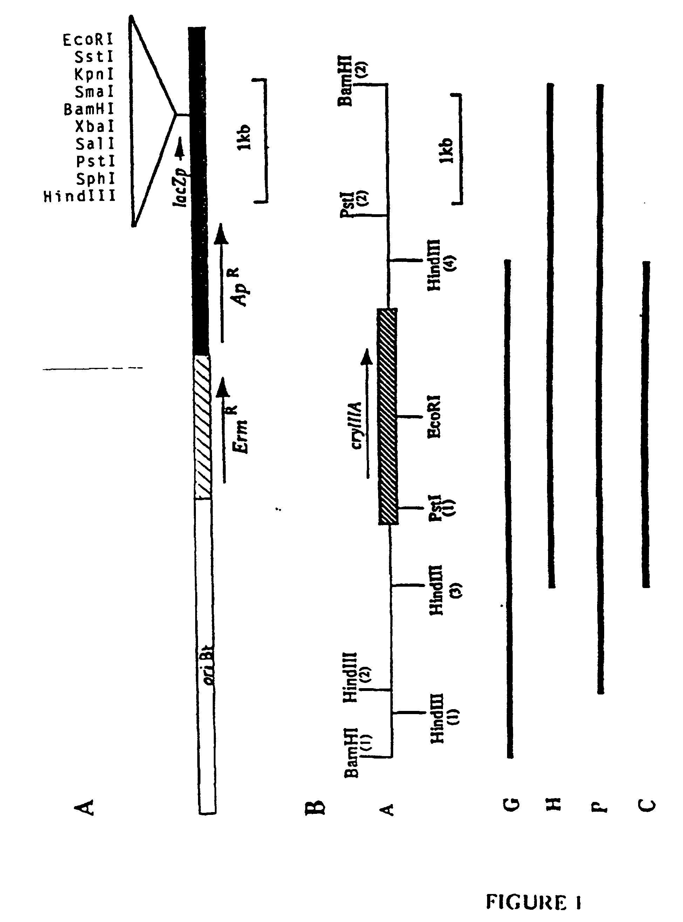

Les sites de restriction dont il est question ci-dessus sont représentés à la figure 1.

Dans la suite du texte on utilisera les abréviations Hₙ pour désigner le site HindIII ayant la position "n" par rapport au premier site HindIII du fragment BamH1. De même l'expression Pₙ désigne le site Pst1 en position "n" par rapport au premier site PstI sur le fragment BamH1.

La séquence d'ADN définie ci-dessus, peut être isolée et purifiée par exemple à partir du plasmide portant le gène cryIIIA de Bacillus thuringiensis.

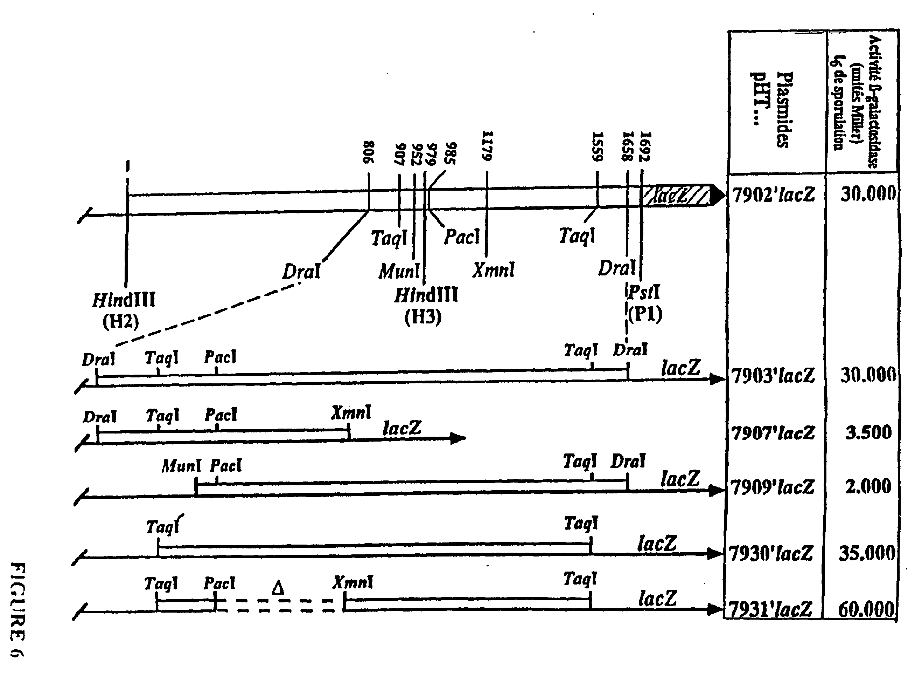

Le système d'expression de CryIIIA comprend une première séquence de nucléotides ou promoteur située entre les sites TaqI et PacI (positiions 907 à 990) et une deuxième séquence de nucléotides ou "région aval" comprise entre les sites XmnI et TaqI (positions 1179 à 1559) tel que démontré à la figure 6. La présence de deux séquences de ce type est préférée pour obtenir un niveau optimal d'expression du gène cryIIIA ou d'un autre gène placé sous le contrôle de ce système d'expression.

Entre également dans le cadre de l'invention, un vecteur d'expression caractérisé en ce qu'il est modifié en l'un de ses sites, par une séquence d'ADN telle que décrite ci-dessus, de façon telle que ladite séquence d'ADN intervient dans le contrôle de l'expression d'une séquence codante de nucléotides déterminée.

Un vecteur de l'invention peut être de préférence un plasmide par exemple un plasmide de type réplicatif.

Un vecteur particulièrement avantageux est le plasmide pHT7902'lacZ déposé à la CNCM (Collection Nationale de Cultures de Micro-organismes - Paris - France) le 20 Avril 1993 sous le N° I-1301.

L'invention a également pour objet un hôte cellulaire recombinant caractérisé en ce qu'il est modifié par une séquence d'ADN telle que définie précédemment, ou par un vecteur d'expression décrit ci-dessus. Un hôte cellulaire particulièrement avantageux est la souche 407-0A::Km^{R} (pHT305P) déposé à la CNCM le 3. mai 1994 sous le n° I-1412.

### Description détaillée de l'invention

L'invention a pour objet une séquence d'ADN susceptible d'influencer l'expression de la partie codante d'un gène dans une cellule hôte bactérienne. Plus particulièrement, l'invention concerne l'association de deux séquences de nucléotides, soit un promoteur et une région aval susceptible d'intervenir au niveau post-transcriptionnel lors de l'expression de la partie codante d'un gène.

Le système d'expression de l'invention qui, comme il sera décrit en détail plus loin, implique vraisemblablement l'hybridation d'une partie de la région aval à l'extrémité 3' de l'ARN 16S d'un ribosome bactérien, peut être utilisé pour l'expression de gènes dans un grand éventail de cellules hôtes. Cette utilisation étendue du système d'expression de l'invention est possible, étant donné l'importante homologie observée au niveau des divers ARN 16S de ribosomes bactériens. Les inventeurs ayant déterminé les régions essentielles à son fonctionnement, le système d'expression de la présente invention peut donc être utilisé dans tout type de bactéries hôtes, les ajustements nécessaires faisant partie des connaissances de l'homme de métier.

De façon générale et sans vouloir s'y restreindre pour des raisons qui deviendront évidentes plus loin, le système d'expression de la présente invention, lorsqu'utilisé pour l'expression de gènes dans des bactéries Gram⁺ de type Bacillus, est situé en amont de la partie codante du gène à exprimer. Plus particulièrement, la région aval est normalement située immédiatement en amont du gène alors que le promoteur est situé en amont de la région aval bien qu'on puisse envisager une autre position pour ce dernier. On peut envisager le déplacement de la région aval lorsque le système est utilisé dans une cellule hôte de type E. coli chez qui les ARNm sont dégradés en sens inverse. On peut également envisager l'utilisation d'une région aval en aval et en amont de la séquence codante, ce qui permettrait la "protection" de la région codante par un mécanisme qui sera décrit en détail plus loin.

Selon un premier mode de réalisation préféré de l'invention, la séquence d'ADN correspond à l'enchaînement HindIII-PstI (H₂-P₁) ci-dessus décrit et comprenant deux séquences de nucléotides (un promoteur et une région aval) ayant des fonctions distinctes.

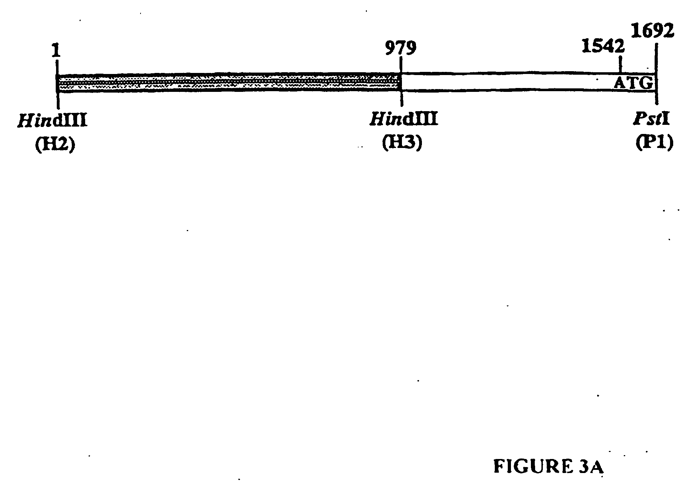

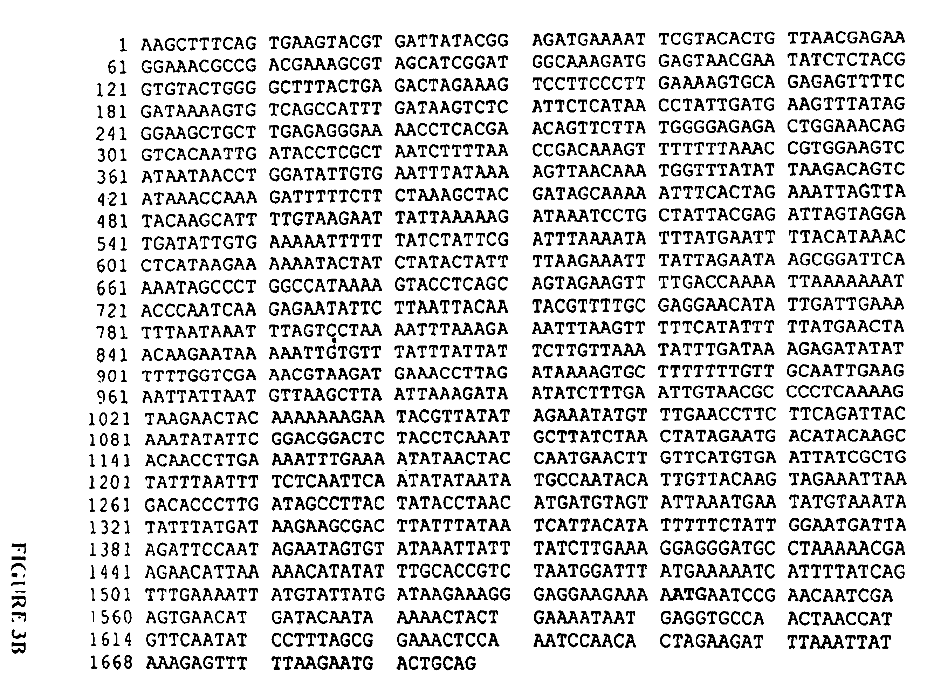

Selon un mode de réalisation particulièrement avantageux de l'invention, la séquence d'ADN correspond à l'enchaînement de nucléotides désignés par l'expression Seq N°1 et correspondant au fragment d'ADN comprenant les nucléotides 1 à 1692 de la séquence représentée à la figure 3.

Le promoteur et la région aval de la séquence d'ADN de l'invention sont décrits en détail ci-dessous. **Séquences de nucléotides présentant une activité promotrice**

De façon préférée, une séquence d'ADN de l'invention intervient au niveau du contrôle de la transcription.

Il s'agit ici des séquences de nucléotides identifiées précédemment comme étant le promoteur. De façon générale et tel que mentionné précédemment, le promoteur se situe en amont de la région aval et donc à une certaine distance de la région codante du gène. Cependant, on peut envisager le déplacement du promoteur tant qu'il reste localisé en amont de la région aval.

Quant à la nature du promoteur, il semble préférable d'utiliser un promoteur provenant de la cellule hôte utilisée pour l'expression du gène d'intérêt. Toutefois, il peut, dans certaines situations, être indiqué d'utiliser un promoteur exogène. Par exemple, des promoteurs tels les promoteurs des gènes degQ, λPL, lacZ, CryI, cryIV ou de l'α-amylène peuvent être utilisés.

Dans le contexte de la présente invention, des fragments particulièrement préférés et comprenant une région promotrice sont les fragments suivants, représentés à la figure 1 :
- l'enchaînement délimité par les sites de restriction TaqI-PaqI ; pour des raisons de commodité de language, on a désigné par PacI l'extrémité de ce fragment qui se trouve en réalité au nucléotide 990 de la séquence de la figure 3 alors que le site PacI se termine en position 985,
- ou tout fragment de cet enchaînement, conservant les propriétés de cet enchaînement vis-à-vis du contrôle de l'expression d'une séquence codante de nucléotides.

Plus particulièrement, toute partie d'au moins 10 nucléotides naturellement consécutifs ou non de cet enchaînement et susceptible d'intervenir dans le contrôle de l'expression d'une séquence codante de nucléotides placée en aval dans un hôte cellulaire constitue une réalisation préférentielle de l'invention. Par exemple, à l'intérieur de la séquence mentionnée précédemment se trouvent les boites -35(TTGCAA) et -10(TAAGCT) du promoteur.

Selon un autre mode de caractérisation de l'invention, les séquences d'ADN "de contrôle" et comprenant le promoteur dont il est question ci-dessus sont caractérisées par leur enchaînement de nucléotides. A cet égard, l'invention a notamment pour objet les séquences d'ADN correspondant aux enchaînements suivants :
- la séquence d'ADN correspondant à l'enchaînement Seq N°3 correspondant au fragment comprenant les nucléotides 907 à 990 de la séquence représentée à la figure 3, ou un variant comprenant les nucléotides 907 à 985.

L'invention a aussi pour objet des séquences d'ADN hybridant dans des conditions non stringentes, telles qu'elles sont définies ci-dessous, avec l'un des enchaînements ci-dessus décrits. Dans ce cas, l'un des enchaînements ci-dessus en question est utilisé comme sonde.

### Séquences de la région aval

Une séquence de l'invention, inclue dans la région aval, est choisie pour sa capacité à intervenir pour augmenter l'expression d'un gène, qui serait initiée par un promoteur situé en amont de cette séquence. Il s'agit vraisemblablement d'une séquence susceptible d'intervenir au niveau post-transcriptionnel lors de l'expression d'une séquence codante.

En effet, les résultats expérimentaux obtenus par les inventeurs semblent indiquer que l'effet post-transcriptionnel de la région aval définie précédemment résulte, à tout le moins lors de l'expression du gène cryIIIA, de l'hybridation entre l'ARN ribosomal 16S de la cellule hôte et une séquence S2 de l'ARN messager de cryIIIA. Il semble que le ribosome ou une partie du ribosome se fixe sur cette région aval et protège ainsi l'ARNm d'une dégradation exonucléasique initiée en 5'. Cette fixation aurait donc pour effet d'augmenter la stabilité des messagers et ainsi d'augmenter le niveau d'expression du gène cloné.

Un des fragments particulièrement préféré dans le cadre de la réalisation de l'invention et pouvant être utilisé à titre de région aval est le fragment suivant, représenté à la figure 1 :
- l'enchaînement délimité par les sites de restriction XmnI-TaqI (positions 1179 à 1556),
- ou tout fragment de cet enchaînement conservant les propriétés de cet enchaînement vis-à-vis du contrôle de l'expression d'une séquence codante de nucléotides.

Selon un autre mode de caractérisation de l'invention, les séquences d'ADN "de contrôle" comprenant la région aval dont il est question ci-dessus sont caractérisées par leur enchaînement de nucléotides. A cet égard, l'invention a notamment pour objet les séquences d'ADN correspondant aux enchaînements suivants :
- la séquence d'ADN correspondant à l'enchaînement Seq N° 4 correspondant au fragment comprenant les nucléotides 1179 à 1559 de la séquence représentée à la figure 3,
- la séquence d'ADN correspondant à l'enchaînement Seq N° 5 correspondant au fragment comprenant les nucléotides 1179 à 1556 de la séquence représentée à la figure 3,
- la séquence d'ADN correspondant à l'enchaînement Seq N° 11 correspondant au fragment comprenant les nucléotides 1413 à 1556 de la séquence représentée à la figure 3,
- la séquence d'ADN correspondant à l'enchaînement Seq N° 8 correspondant au fragment comprenant les nucléotides 1413 à 1461 de la séquence représentée à la figure 3,
- la séquence d'ADN correspondant à l'enchaînement Seq N° 9 correspondant au fragment d'ADN suivant : 
5'-AGCTTGAAAGGAGGGATGCCTAAAAACGAAGAACTGCA-3'
3'-ACTTTCCTCCCTACGGATTTTTGCTTCTTG-5'
- la séquence d'ADN correspondant à l'enchaînement Seq N° 10 correspondant au fragment d'ADN suivant :

5'-CTTGAAAGGAGGGATGCCTAAAAACGAAGAAC-3'
3'-GAACTTTCCTCCCTACGGATTTTTGCTTCTTG-5'

L'invention a aussi pour objet des séquences d'ADN hybridant dans des conditions non stringentes, telles qu'elles sont définies ci-dessous, avec l'un des enchaînements ci-dessus décrits. Dans ce cas, l'un des enchaînements ci-dessus en question est utilisé comme sonde.

Il semble que la région aval comprenne, dans un premier temps, une région dite "essentielle", suffisamment complémentaire à l'extrémité 3' d'un ARN ribosomal 16S bactérien pour permettre la fixation du ribosome sur cette région essentielle. En aval de cette région essentielle portant le site de fixation du ribosome, se situerait une deuxième région, comprenant une structure supplémentaire susceptible d'avoir un effet positif additionnel au niveau de l'expression de la séquence codante. Il est possible que cette deuxième séquence empêche le mouvement du ribosome une fois ce dernier fixé sur la région essentielle.

Par exemple, dans le système d'expression du gène CryIIIA, il semble que la séquence de nucléotides située entre les positions 1413 et 1556 de la séquence représentée à la figure 3 comprenne la région essentielle de fixation au ribosome ainsi que la deuxième région en aval du site de fixation. Bien que la deuxième région ne soit pas absolument essentielle pour obtenir une expression accrue de la séquence codante, il semble que sa suppression réduise les rendements d'expression. En effet, des résultats expérimentaux ont démontré que la suppression de la région se situant entre les nucléotides 1462 et 1556 de la séquence représentée à la figure 3 entraîne une légère diminution de l'expression de la séquence codante.

Il semble que la longueur minimale de la séquence de nucléotides permettant une fixation adéquate du ribosome soit d'environ 10 acides nucléiques. L'invention a donc également pour objet toute partie d'au moins 10 nucléotides naturellement consécutifs ou non de l'enchaînement H₂-P₁, susceptible de contrôler dans un hôte cellulaire de type Bacillus, l'expression d'une séquence codante de nucléotides placés en aval de cette partie de l'enchaînement H₂-P₁.

Dans le cas spécifique du système d'expression du gène CryIIIA, il semblerait que la séquence de la région "essentielle" incluant le site de fixation soit la suivante :

5'-GAAAGGAGG-3'
3'-CTTTCCTCC-5'

Il est possible d'effectuer des modifications mineures au site de fixation dans la mesure où l'intensité de l'interaction entre l'extrémité 3' de l'ARN 16S du ribosome et cette région "essentielle" soit suffisamment forte pour qu'il y ait hybridation entre le ribosome et le site de fixation. A partir de calculs d'énergie d'interaction qui peuvent être effectués par l'homme de métier, des modifications au site de fixation peuvent être envisagées si l'intensité de la liaison demeure à peu près semblable à l'intensité mesurée lors de l'utilisation de la région "essentielle" naturelle.

Dans le cas du site de fixation illustré précédemment, il est possible d'envisager certaines modifications aux quatre premiers nucléotides ainsi qu'au septième nucléotide. Il semble toutefois que les nucléotides en positions 5, 6, 8 et 9 soient importants pour assurer une intensité d'interaction appropriée lors de l'hybridation à l'ARN 16S du ribosome.

L'extrémité 3' de l'ARN 16S de ribosome bactérien étant relativement bien conservée d'une espèce bactérienne à l'autre, le système d'expression de la présente invention peut donc être utilisé dans un grand nombre d'hôtes bactériens sans avoir à effectuer de modifications substantielles.

L'invention a donc également pour objet une séquence d'ADN caractérisée par les propriétés suivantes :
- elle est contenue dans un enchaînement de nucléotides hybridant dans des conditions non stringentes avec le fragment d'ADN compris entre les nucléotides 1413 et 1559 de la séquence représentée à la figure 3 ;
- elle est susceptible d'intervenir dans le contrôle de l'expression dans une cellule hôte d'une séquence codante, notamment d'une séquence codant pour un polypeptide de Bacillus, toxique vis-à-vis des insectes ou d'une séquence codant pour un polypeptide exprimé pendant la phase stationnaire de Bacillus.

Une séquence codant pour un polypeptide de Bacillus, toxique vis-à-vis des larves d'insectes est par exemple une séquence comprise dans le gène cryIIIB de B. thuringiensis.

Une séquence d'ADN répondant à cette définition peut être identifiée en utilisant des amorces oligonucléotidiques.

Une hybridation en conditions non stringentes entre la séquence d'ADN testée et le fragment d'ADN compris entre les nucléotides 1413 et 1559 de la séquence de la figure 3 utilisée comme sonde sera effectuée comme suit :
La sonde ADN et les séquences fixées sur filtre de nitrocellulose ou sur filtre nylon sont hybridées à 42°C pendant 18 H avec agitation, en présence de formamide (30 %), de SSC 5X de la solution de Denhardt 1X. La solution de Denhardt 1X est composée de 0,02 % de ficoll, de 0,02 % de polyvinyl pyrrolidone et de 0,02 % de sérum albumine bovine. Le SSC 1X est composé de NaCl 0,15 M et de citrate de sodium 0,015 M. Après hybridation, le filtre est successivement lavé à 42°C pendant 10 minutes dans chacune des solutions suivantes :
   - Formamide (30 %), SSC 5X
   - SSC 2X
   - SSC 1X
   - SSC 0,5X

Les conditions d'hybridation décrites précédemment sont celles qui sont utilisées pour toutes les applications de la présente invention lorsque nécessaire.

Les séquences d'ADN selon l'invention peuvent être, le cas échéant, recombinées entre elles ou associées sur le vecteur en des sites différents. En particulier, on associera avantageusement sous forme d'un enchaînement unique, le fragment TaqI-PacI au fragment XmnI-TaqI définis ci-dessus ou encore le fragment TaqI-PacI à l'enchaînement Seq N° 8. De telles séquences ont la propriété avantageuse de permettre un haut niveau d'expression (jusqu'à 60.000 unités Miller) de la séquence codante de nucléotides, niveau d'expression qui peut être constaté avec le gène de la β-galactosidase .

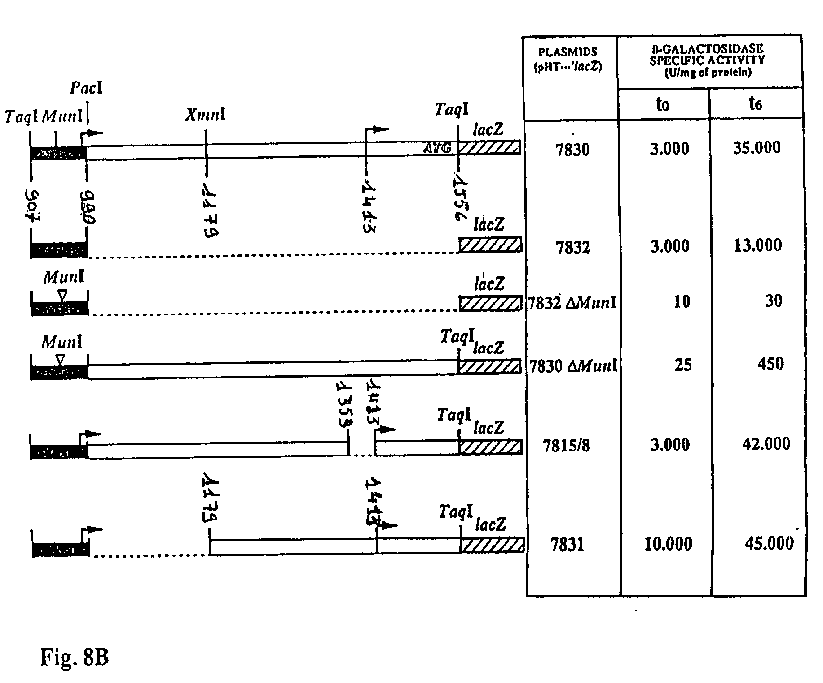

De plus, des fragments particulièrement préférés dans le cadre de la réalisation de l'invention sont les fragments suivants représentés à la figure 8B :
- l'enchaînement délimité par les sites de restriction TaqI-TaqI,
- ou tout fragment de ces enchaînements conservant les propriétés de ces enchaînements vis-à-vis du contrôle de l'expression d'une séquence codante de nucléotides.

Selon un autre mode de caractérisation de l'invention, les séquences d'ADN dont il est question ci-dessus sont caractérisées par leur enchaînement de nucléotides. A cet égard, l'invention a notamment pour objet les séquences d'ADN correspondant aux enchaînements suivants :
- L'enchaînement Seq N° 2, correspondant au fragment comprenant les nucléotides 907 à 1559 de la séquence représentée à la figure 3,
- la séquence d'ADN correspondant à l'enchaînement Seq N° 6 correspondant au fragment comprenant les nucléotides 907 à 1353 et 1413 à 1556 de la séquence représentée à la figure 3,
- la séquence d'ADN correspondant à l'enchaînement Seq N° 7 correspondant au fragment comprenant les nucléotides 907 à 990 et 1179 à 1559 de la séquence représentée à la figure 3.

L'invention a aussi pour objet des séquences d'ADN hybridant dans des conditions non stringentes, telles qu'elles sont définies ci-dessus, avec l'un des enchaînements ci-dessus décrits. Dans ce cas, l'un des enchaînements ci-dessus en question est utilisé comme sonde.

Les séquences d'ADN de l'invention peuvent être isolées et purifiées à partir de Bacillus, notamment de B. thuringiensis ; elles peuvent être préparées également par synthèse selon les techniques connues.

Entrent également dans le cadre de l'invention, les séquences d'ARN correspondant aux séquences d'ADN ci-dessus décrites.

L'invention a également pour objet une séquence d'ADN recombinant caractérisée en ce qu'elle comprend une séquence codante déterminée, sous le contrôle d'une séquence d'ADN répondant à l'une des définitions précédentes.

La capacité des ADN de l'invention à intervenir dans le contrôle de l'expression de séquences de nucléotides, peut être vérifiée par la mise en oeuvre du test suivant :
- la séquence d'ADN de l'invention dont on veut évaluer la capacité à intervenir dans le contrôle de l'expression d'une séquence codante est insérée dans un plasmide à faible nombre de copies, en amont d'une séquence codante de nucléotides.
- le plasmide ainsi préparé est utilisé pour transformer (par exemple par électroporation) une souche de Bacillus thuringiensis et par exemple une souche B. thuringiensis HD1 cry⁻ B ;
- la souche de bacillus ainsi transformée est cultivée dans des conditions permettant l'expression de la séquence codante de nucléotides ;
- on détecte le produit d'expression de cette séquence codante de nucléotides, par des techniques de mesure qualitative et/ou quantitative courantes.

Pour la réalisation de ce test, la séquence codante de nucléotides sera avantageusement la séquence codante du gène cryIIIA de Bacillus thuringiensis ou, par exemple une séquence codant pour la *β-*galactosidase.

### Hôtes cellulaires

Différents types d'hôtes cellulaires peuvent être mis en oeuvre dans le cadre de l'invention. On citera à titre d'exemple Bacillus, par exemple Bacillus thuringiensis ou Bacillus subtilis. On peut également envisager d'utiliser des cellules telles que E. coli.

Dans des hôtes cellulaires capables de sporuler, la séquence codante peut être exprimée pendant la phase végétative ou la phase stationnaire de croissance ou pendant la sporulation.

Un hôte cellulaire intéressant dans le cadre de l'invention peut également être constitué par une cellule végétale ou animale.

Si cela est nécessaire ou souhaité, selon la nature de la séquence codante de nucléotides exprimée, une séquence signal peut être également insérée dans le vecteur d'expression de l'invention afin que le produit d'expression de la séquence codante soit exposé à la surface de l'hôte cellulaire, voire exporté, à partir de cet hôte cellulaire.

De façon tout à fait intéressante, on pourra utiliser des souches asporogènes de bacillus soit naturellement, soit par mutation, et en particulier des souches de Bacillus subtilis ou Bacillus thuringiensis.

Les inventeurs ayant mis en évidence que les séquences d'ADN de l'invention permettent l'expression d'une séquence codante déterminée indépendamment de la phase de sporulation de souches de type Bacillus, un hôte asporogène peut présenter l'avantage de fournir des moyens d'expression de séquences codantes susceptibles d'entrer dans des compositons biopesticides dont les effets négatifs éventuels vis-à-vis de l'environnement seraient atténués, voire supprimés.

L'hôte asporogène choisi est particulièrement avantageux pour exprimer une séquence codante pendant sa phase stationnaire de croissance, quand la séquence codante est sous le contrôle d'une des séquences de l'invention.

Dans le cas de souches asporogènes de bacillus obtenues par mutation, un exemple illustrant l'efficacité particulière de ce type de souche pour l'expression d'une séquence codante pendant la phase stationnaire de croissance est la construction d'une souche de B.thuringiensis mutée pour le gène spoOA. Une souche de B.thuringiensis dont le gène spoOA est inactivé et portant un gène, par exemple un gène de toxine insecticide CryI, cryII, cryIII ou cryIV ou encore un gène d'intérêt industriel, dont l'expression est mise sous le contrôle du système d'expression de CryIIIA, présente des caractéristiques avantageuses. Notamment la souche B.thuringiensis 407.OA::Km^{R} (pHT305P) dont la construction est décrite en détail plus loin a au moins les avantages suivants :
a) surproduction de protéines pendant la phase stationnaire de croissance ;
b) les protéines (par exemple des biopesticides) restent incluses dans la cellule et auraient ainsi une persistance accrue dans l'environnement ; et
c) les problèmes potentiels liés à la dissémination de spores sont ainsi évités.

D'autres caractéristiques et avantages de l'invention découlent des exemples qui suivent ainsi que des figures :
Figure 1 : Carte de restriction schématique des plasmides utilisés
   (A) - Carte physique du vecteur navette pHT304 Les flèches au-dessus Erm^{R} et Ap^{R} indiquent la direction de transcription des gènes ermC et bla respectivement. La flèche et l'expression LacZp indiquent la direction de transcription à partir du promoteur du gène LacZ. ori Bt est la région de réplication du plasmide pHT1030 de B. thuringiensis. (B) - Carte de restriction simplifiée des fragments comportant le gène cryIIIA. Le fragment A est un fragment de 6 kb BamHI. de B. thuringiensis LM79 ; les fragments de restriction G, P et H ont été obtenus à partir de digestion partielle par HindIII et C a été obtenu après digestion totale avec HindIII à partir du fragment A. Ces fragments ont été clonés dans pHT304 pour donner les dérivés pHT305A, pHT305G, pHT305P, pHT305H et pHT305C respectivement. Le gène cryIIIA (cadre hachuré) et la direction de transcription sont indiqués. Les nombres sous chaque site indiquent leur ordre de la gauche vers la droite.
Figure 2 : Analyse des protéines des transformants de B. thuringiensis exprimant le gène CryIIIA. Un volume identique d'échantillons (20 µl) a été chargé dans chaque puits. Les lignes 1 à 4 et 6 à 8 de B. thuringiensis Kurstaki HD1 Cry⁻B portant pHT305A, pHT305G, pHT305H, pHT305P, pHT305HΩH₂-H₃, pHT305C et pHT304 respectivement. La ligne 5 correspond aux marqueurs de poids moléculaire (du haut vers le bas 97, 66, 60, 43 et 30 kDa). Les flèches indiquent les composants du cristal de 73 et 67 kDa.
Figure 3 : Séquence de nucléotides de l'extrémité 5' de la région en amont du gène cryIIIA.
   (A) Carte physique du fragment H₂-P₁ (H₂-H₃+H₃-P₁) dans l'orientation 5' vers 3'. Les positions des nucléotides des deux sites HindIII (H₂ et H₃) qui délimitent le fragment grisé, sont indiquées. Le second segment séquencé (fragment H₃-P₁) était le fragment entre le troisième site HindIII et le site Pst1 (P₁). Un site ATG d'initiation de transcription de la toxine CryIIIA est montré. La numérotation des nucléotides est rapportée par rapport au fragment séquencé et non par rapport à l'initiation de transcription.
   (B) - Séquence de nucléotides du fragment H₂-P₁. Le codon d'initiation ATG est indiqué en caractères gras et l'extrémité du transcrit majoritaire sur gel, spécifique du gène cryIIIA, correspond au T localisé en position 1413. Un autre transcrit commence au nucléotide 983 ; il est apparemment minoritaire sur gel. La séquence comporte au moins deux répétitions inversées. La numérotation des nucléotides débute à partir du second site HindIII et se termine au site Pst1 montré sur la figure 3A.

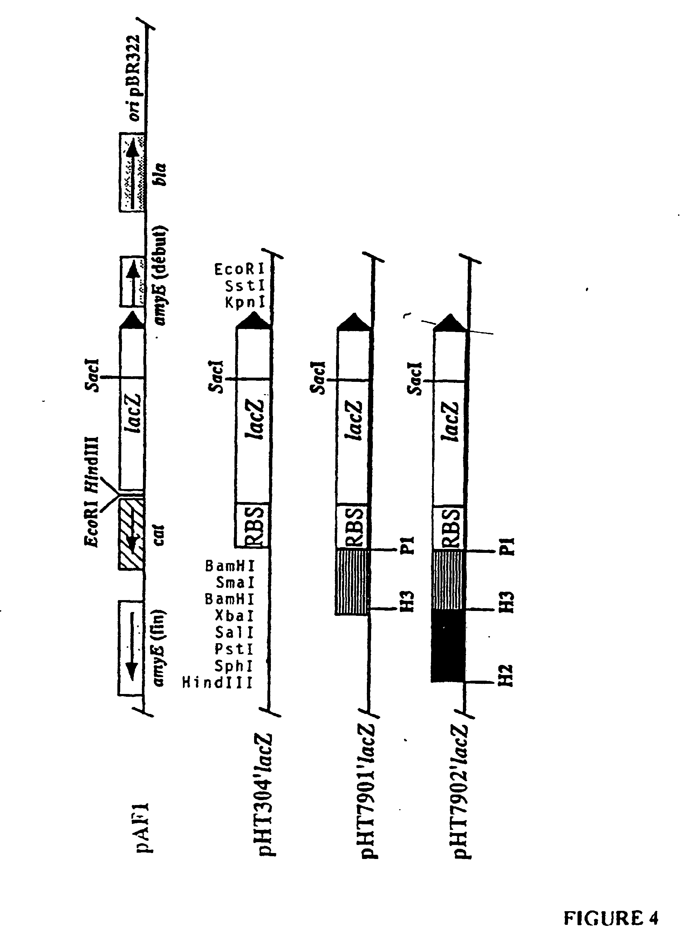

Figure 4 : Représentation des plasmides PAF1, pHT304'lacZ, pHT7901'lacZ et pHT7902'lacZ.

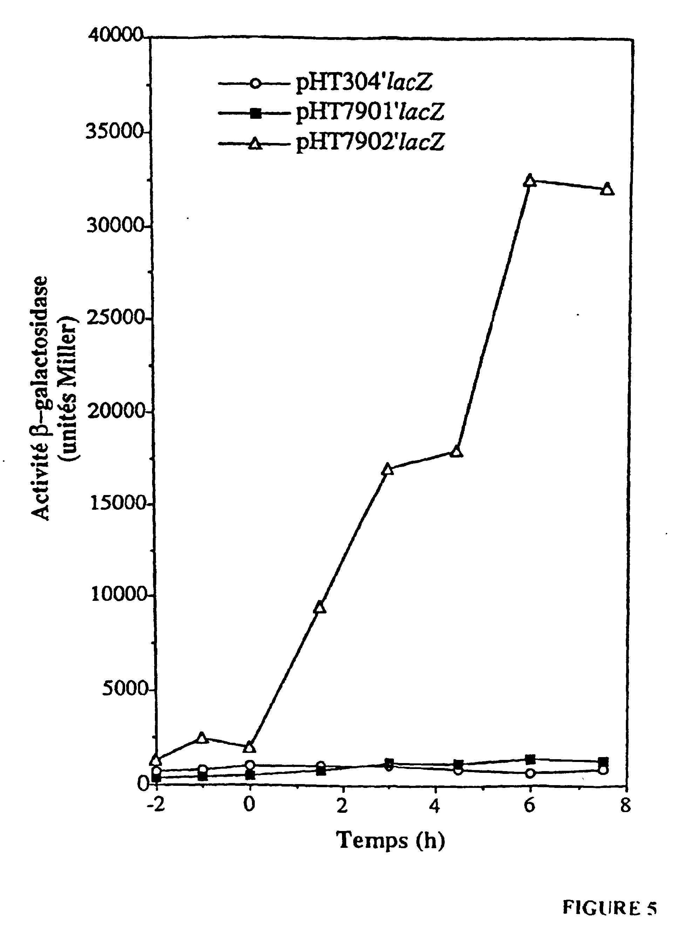

Figure 5 : Profil d'activité β-galactosidase. La croissance des cellules Bt et les conditions de réalisation des échantillons ainsi que le test sont décrits dans "Matériel et Méthodes". Le temps t₀ indique la fin de la phase exponentielle et tₙ est le nombre d'heures avant (-) ou après le temps zéro.
Figure 6 : Carte de restriction détaillée des plasmides pHT7902'lacZ, 7903'lacZ, 7907'lacZ, 7909' lacZ, 7930' lacZ et 7931'lacZ. Ces plasmides ont été insérés dans B. thuringiensis et l'activité β-galactosidase a été mesurée au temps t₆ de sporulation (en unités Miller). On observe des activités respectivement de 30.000, 30.000, 3.500, 2.000, 35.000 et 60.000.

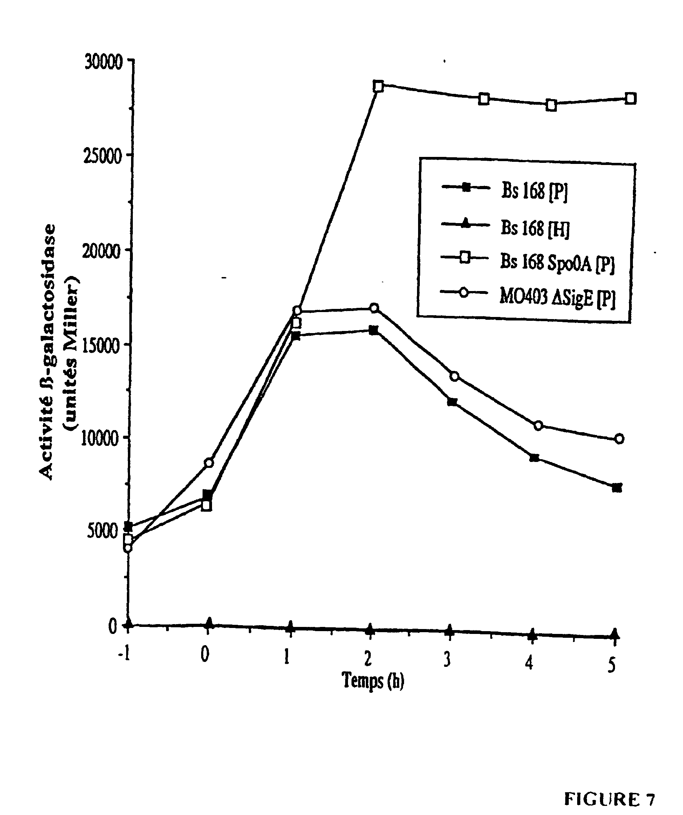

Figure 7 : Activité *β*-galactosidase dans des souches de B. subtilis Spo⁻ et Spo⁺ ; les cultures sont faites en milieu SP.

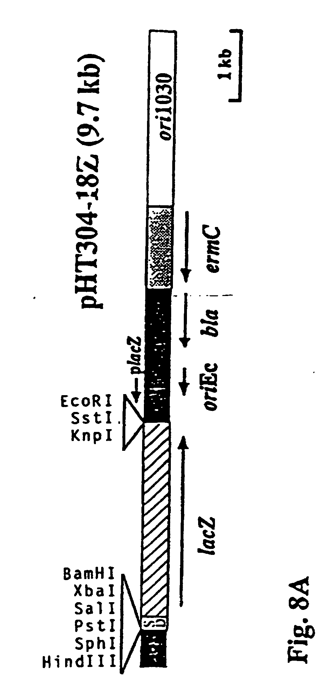

Figure 8 : Carte de restriction schématique des constructions utilisées pour mesurer l'activité transcriptionnelle des régions du système d'expression de cryIIIA, chez B.thuringiensis souche kursrtaki HD1 Cry⁻B.
   A - Carte physique du vecteur pHT304-18Z. Les flèches indiquent la direction de transcription des gènes ermC, bla, lacZ et du promoteur placZ; et l'orientation de la réplication chez E.coli (oriEc). ori1030 indique la région de réplication du plasmide pHT1030 (Lereclus et Arantes, Mol. Microbiol. 1992, 7:35-46).SD indique le site de fixation des ribosomes du gène spoVG placé devant le gène lacZ (Perkins et Youngman, 1986, Proc. Natl. Acad. Sci. USA, 83:140-144).
   B - Représentation physique et activité transcriptionnelle des différentes régions du système d'expression de cryIIIA fusionnées avec le gène lacZ. La numérotation des nucléotides est établie d'après la séquence d'ADN du fragment H2-P1 présentée Fig. 3B. Les flèches indiquent la position des extrémités 5' des transcrits telles qu'elles sont identifiées par extension d'amorce. Les traits en pointillé indiquent la localisation des fragments délétés. L'activité β-galactosidase des différentes constructions a été mesurée aux temps t₀ et t₆ de la sporulation et est indiquée en unités Miller.

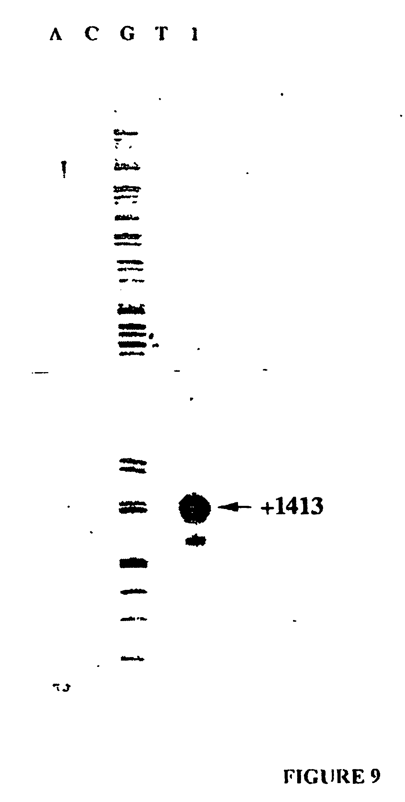

Figure 9 : Détermination de l'extrémité 5' du transcrit cryIIIA/lacZ produit par la souche de B.thuringiensis portant le plasmide pHT7815/8'lacZ. L'ARN total des cellules a été extrait à t₃ et soumis à une expérience d'extension d'amorce à la transcriptase inverse en utilisant comme amorce l'oligonucléotide suivant : 5'-CGTAATCTTACGTCAGTAACTTCCACAG>-3'. Cet oligonucléotide est complémentaire de la région localisée entre le site de fixation des ribosomes du gène spoVG et le codon d'initiation du gène lacZ. Le même oligonucléotide a été utilisé pour déterminer la séquence nucléotidique de la région correspondante du plasmide pHT7815/8. L'extrémité 5' est numérotée d'après la séquence d'ADN du fragment H2-P1 présentée Fig. 3B.

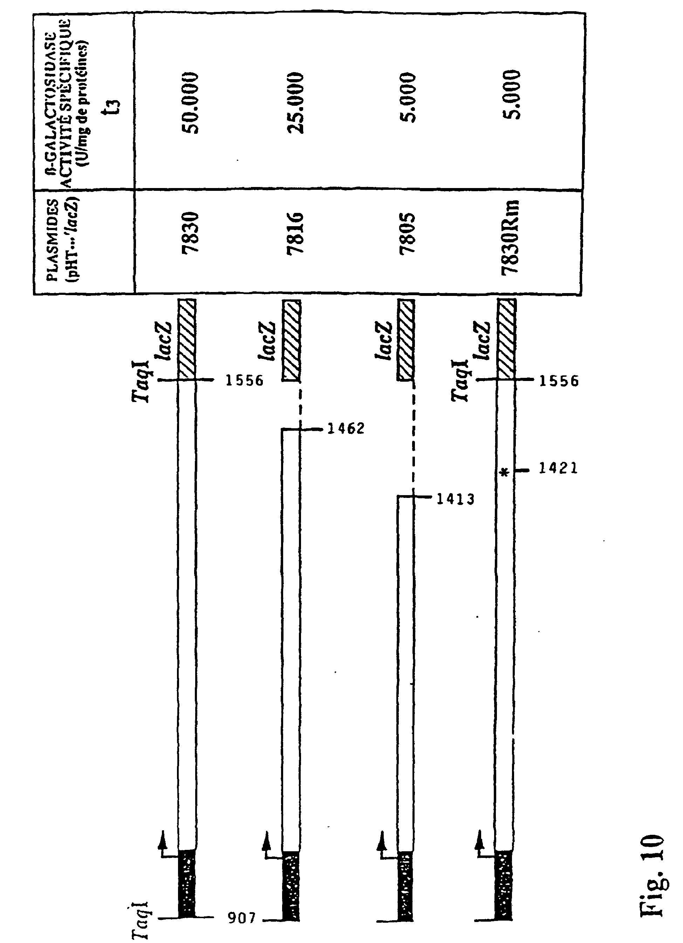

Fig. 10 : Carte physique schématique des constructions utilisees pour mesurer l'activité post-transcriptionnelle de la région aval du système d'expression de cryIIIA, chez B.subtilis souche 168. La numérotation des nucléotides est établie d'après la séquence d'ADN du fragment H2-P1 présentée Fig. 3B. La flèche indique la position du démarrage de transcription localisé en position +984. L'astérisque en position 1421 indique le remplacement GGA par CCC. Les traits en pointillé indiquent la localisation des fragments d'ADN délétés. L'activité β-galactosidase des différentes constructions a été mesurée au temps t₃ de la sporulation et est indiquée en unités Miller.

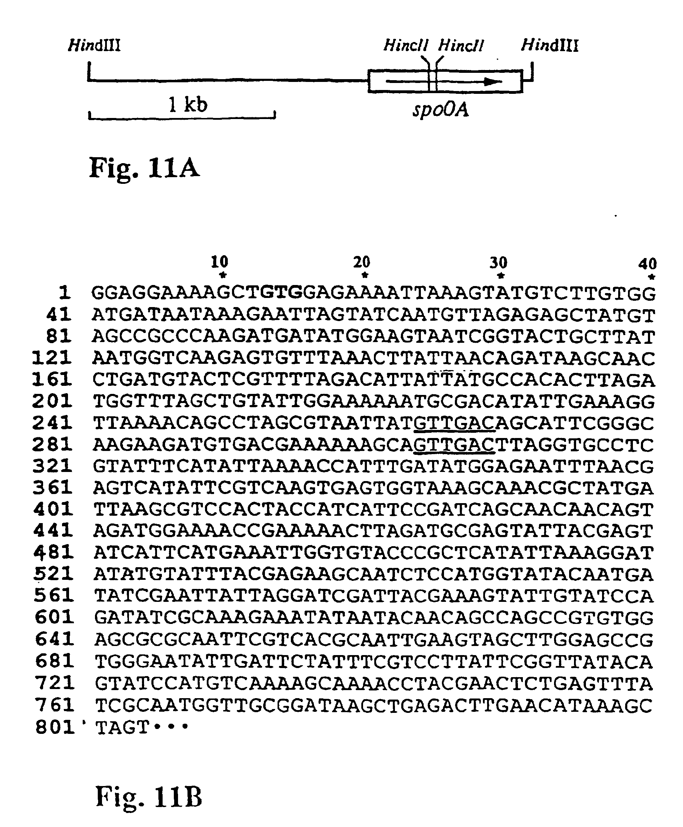

Figure 11 : Séquence de nucléotides du gène spoOA de B.thuringiensis souche 407.
   A - Carte de restriction schématique du fragment d'ADN de 2,4 kb portant le gène spoOA. La flèche indique l'orientation de la transcription du gène spoOA.
   B - Séquence nucléotidique de la phase ouverte de lecture comportant la séquence codante du gène spoOA. Le codon d'initiation GTG est indiqué en caractère gras. Les deux sites HincII sont soulignés. Les trois points représentent le codon stop.

### Matériel et méthodes

### SOUCHES BACTERIENNES ET CONDITIONS DE CROISSANCE

Escherichia coli K-12 TG1 [Δ(lac-proAB)supE thi hdsD (F' traD36 proA⁺ proB⁺ lacI^{q} lacZΔDM15)] Gibson, T.J. et al 1984. thesis. University of Cambridge, Cambridge a été utilisé en tant qu'hôte pour la construction des plasmides représentés à la figure 1B et pour le bactériophage M13.
E. coli MC1061 [hsdR mcrB araD139Δ (araABC-leu) 7679 Δ lacX74 galU galK rpsL thi] (Meissner, P.S. et al 1987. Proc. Natl. Acad. Sci.USA 84:4171-4175) a été utilisé en tant qu'hôte pour la construction des plasmides représentés à la figure 7.
B. thuringiensis souche LM 79 qui contient le gène cryIIIA, a été isolé et caractérisé par Chaufaux J. et al 1991. Les colloques de l'INRA. 58:317-324. Cette souche appartient au sérotype 8 et produit des quantités de toxines similaires à celles produites par d'autres souches de B thuringiensis portant le gène cryIIIA (Donovan, V.P., et al 1988. Mol. Gen. Genet. 214.365-372. - Sekar, V., et al 1987. Proc. Natl. Acad. Sci. USA 84:7036-7040).
B. thuringiensis de la sous-espèce Kurstaki HD1 Cry⁻ B a été utilisé comme hôte pour les études de régulation du gène cryIIIA. Les souches E. coli ont été cultivées à 37°C dans un milieu Luria et transformées selon la méthode décrite par Lederberg et Cohen (1974. Bacteriol. 119:1072-1074).
La souche B thuringiensis espèce Kurstaki HD1 Cry⁻B a été cultivée et transformée par électroporation selon la technique décrite par Lereclus et al, (1989. FEMS Microbiol. Lett. 60:211-218).
Les concentrations en antibiotiques pour la sélection des bactéries était de 100 µg/ml pour l'ampicilline et 25 µg/ml pour l'érythromycine.

### Constructions de plasmides

Le fragment BamHI de 6 Kb portant le gène cryIIIA et les régions adjacentes a été isolé à partir de B. thuringiensis LM79 et a été inséré dans le site unique BamHI de pUC19 pour produire pHT791, qui a été employé comme source d'ADN pour la construction des différents plasmides utilisés ici. Le plasmide pHT305A a été obtenu par insertion du fragment BamHI de 6 Kb dans le site unique BamHI du vecteur navette pHT304 (Arantes, O et Lereclus D. 1991, Gene 108:115-119) (Figure 1 A). Des échantillons du fragment BamHI de 6 Kb ont été partiellement ou complètement digérés avec HindIII et les fragments résultants ont été clonés entre les sites BamHI et HindIII ou au site HindIII de pHT304 pour donner les dérivés pHT305G, pHT305H, pHT305P et pHT305C (Figure 1). Le plasmide pHT305HΩH₂H₃ a été obtenu en insérant le fragment H₂-H₃ rempli aux extrémités dans le site SmaI de pHT305H (fragment délimité respectivement par les deuxième et troisième sites HindIII du fragment de 6 kb).

Le fragment de 4,5 Kb SmaI - KpnI du plasmide pTV32 (Perkins, J.B. et al 1986. Proc. Natl. Acad. Sci. USA 83:140-144) contenant les gènes lacZ ermC a été clone dans pEB111 (Leonhardt, H., et al 1988. J. Gen. Microbiol. 134:605-609) pour donner le plasmide pMC11. Le plasmide pHT304'lacZ, utilisé pour construire les fusions de transcription, a été obtenu en clonant le fragment de restriction DraI-SmaI de 3,2 Kb contenant le gène lacZ dépourvu de promoteur, isolé à partir de pMC11, au site unique SmaI de pHT304. Le plasmide pHT7901'lacZ a été obtenu en clonant le fragment H₃-P₁ [(HindIII-PstI) voir figure 3A] entre les sites uniques HindIII et PstI de pHT304'lacZ. Le plasmide pHT7902'lacZ a été construit en clonant le fragment H₂-H₃ (figure 3A) dans le site unique HindIII de pHT7901'lacZ. L'orientation du fragment H₂-H₃ a été déterminée en cartographiant les sites de restriction HpaI et BalI par rapport au site PstI. Deux sites HpaI sont localisés aux positions nucléotidiques 50 et 392 ; le site BalI est localisé à la position nucléotidique 670 (figure 3). La structure générale des plasmides recombinants portant la fusion lacZ est donnée à la figure 4.

### Manipulations d'ADN

Les procédures standard ont été utilisées pour extraire les plasmides à partir de E. coli, pour transfecter l'ADN recombinant du phage M13 et pour purifier l'ADN simple brin (Sambrook J, et al 1989. A laboratory manual, 2nd ed. Cold Spring Harbor Laboratory - Cold Spring Harbor, N.Y.). Les enzymes de restriction, la ligase ADN T4 et la T4 polynucléotide kinase ont été utilisées selon les recommandations du fabricant. Le fragment de Klenow de l'ADN polymérase I et des désoxyribonucléosides triphosphates ont été utilisés pour que les extrémités du fragment H₂-H₃ soient des extrémités franches. Les fragments de restriction d'ADN ont été purifiés sur gels d'agarose en utilisant le kit Prep A gene (Bio-Rad). Les séquences de nucléotides ont été déterminées par la méthode didéoxy de terminaison de chaîne (Sanger, F., et al 1977. Proc. Natl. Acad. Sci. Vol. 175, 1993 USA 74:5463-5467) en utilisant les phages M13mp18 et M13mp19 comme matrices, ainsi que le kit Séquenase version 2.0 (US Biochemical. Cor. Cleveland Ohio) et [α-³⁵S] dATP (15 TBq ; Amersham, United Kingdom).

### Analyse informatique

Les séquences d'ADN ont été analysées en utilisant les programmes de l'Institut Pasteur sur un ordinateur général de données MV10000.

### Extraction de l'ARN, Extension des amorces, Analyse Northern de l'ARN et analyse en dot blot

La souche B. thuringiensis sous espèce Kurstaki HDl Cry⁻B (pHT305P) a été cultivée dans un milieu HCT (Lecadet et al 1980. J. Gen. Microbiol. 121:203-212) à 30°C en agitant. Les échantillons ont été pris à t₀, t₃, t₆ et t₉ (t₀ est défini comme étant le début de la sporulation et tₙ indique le nombre d'heures après le début de la sporulation). Les cellules ont été récupérées par centrifugation, resuspendues dans un milieu HCO (Lecadet, M. M., et al 1980. J. Gen. Microbiol. 121:203-212) contenant 50 mM d'azide de sodium et immédiatement congelées à - 70°c jusqu'à l'extraction d'ARN (Glatron, M.F., et al 1972. Biochimie 54:1291-1301). Pour le test d'allongement de l'amorce, un premier oligonucléotide de 39 mer (3'-CTT AGG CTT GTT AGC TTC ACT TGT ACT ATG TTA TTT TTG-5') complémentaire de la région 3'-1544 à 1583-5'du gène cryIIIA a été synthétisé et son extrémité 5' a été marquée avec du [γ-³²P] dATP (110 TBq/mmol) par la T4 polynucléotide kinase. L'oligonucléotide de 39-mer a été purifié sur une colonne Sephadex G-25 (Pharmacia) (incorporation d'environ 70 %) et pour être utilisé comme amorce, il a été mélangé avec 50 µg d'ARN total.

Un deuxième oligonucléotide de 32 mer, complémentaire la région localisée entre les positions 1090 et 1121, a été utilisé également comme amorce et a permis la détection d'un deuxième transcrit dont le démarrage de transcription est situé en position 983. Cet oligonucléotide correspond à la séquence 5'GTTAGATAAGCATTTGAGGTAGAGTCCGTCCG-3'.

L'hybridation (à 30°C), l'extension de l'amorce et l'analyse des produits ont été effectuées comme décrit par Débarbouillé, M., et al (1983. J. Bacteriol. 153:1221-1227). Les amorces de 39 mer et de 32 mer ont été utilisées pour l'allongement du fragment H₃-P₁ cloné dans M13-mp19 et pour l'allongement du fragment H₂-P₁ cloné dans pHT7902'lacZ, respectivement.Les produits résultant des réactions ont été placés sur des gels en parallèle avec des produits de transcription, pour déterminer les extrémités 5' des transcrits.

Une analyse Northern blot a été réalisée avec de l'ARN dénaturé fractionné par électrophorèse sur des gels d'agarose contenant du formaldehyde à 1,5 % et transféré sous vide sur des membranes Hybond-N⁺ (Amersham) dans 20 x SSC pour 1 h (1 x SSC correspond à 150 mM NaCl plus 15 mM citrate sodium, pH 7,0). Le fragment de restriction PstI-EcoRI de 874 bp (interne au gène cryIIIA) a été marqué avec du ³²P avec un kit de translation de coupure (Boehringer Mannheim), puis dénaturé et utilisé comme sonde. Une préhybridation a été réalisée à 42°c pendant 4 heures dans un milieu contenant 50 % de formamide-1M NaCl-1% sodium dodecyl sulfate (SDS)-10 x Denhardt's solution-50 mM Tris-HCl (pH 7,5)-0,1 % sodium PP, ADN de sperme de saumon dénaturé (≥100 µg/ml) et la sonde marquée (10⁸cpm/µg) a été ajoutée à la solution de préhybridation et l'incubation a été poursuivie pendant toute la nuit. La membrane a été lavée à 65°c pendant 30 minutes 2 fois dans 2 x SSC-0,5 % SDS, une fois dans 2 x SSC-0,5 % SDS, et une fois dans 0,5 x SSC-0,5 % SDS.

Des quantités égales d'ARN de cultures synchrones de B. thuringiensis sous espèce Kurstaki HD1 Cry⁻B portant les plasmides pHT305P ou pHT305H prises à t₃ ont été déposées sur des membranes Hybond-C Extra (Amersham) avec un appareil manifold (Schleicher & Schuel) en utilisant le protocole dot blot décrit par Sambrook et al (Sambrook, J., et al 1989. Cold Spring Harbor Laboratory, Cold Spring Harbor, N.Y.). La sonde et les conditions d'hybridation étaient celles décrites dans les tests Northen blot.

### Préparation du cristal et analyse

Les cellules ont été cultivées dans un milieu HCT à 30°C avec agitation pendant 48 heures et les cristaux ont été préparés selon la méthode décrite dans la publication Lecadet, M.M., et al (1992. Appl. Environ. Microbiol. 58:840-849), à l'exception du fait que la concentration en NaCl était de 150 mM. Pour l'électrophorèse en gel de polyacrylamide-SDS (PAGE), 20 µl de chaque échantillon ont été utilisés (Lereclus, D., et al 1989. (FEMS Microbiol. Lett. 66:211-218).

### Test de détection de la β-Galactosidase

Les souches de E. coli et B. thuringiensis contenant les fusions de transcription lacZ ont été détectées en déposant sur le milieu solide, le substrat chromogène, du 5-bromo-4-chloro-3-indolyl-β-D-galactopyranoside (X-Gal) (40 µg/ml) et des antibiotiques appropriés. Les souches isolées ont été cultivées comme indiqué et récupérées à t₋₂, t₋₁, tₒ, t_{1,5}' t₃, t_{4,5}, t₆, et t_{7,5} .Après centrifugation, les culots ont été immédiatement congelés à - 70°C (pour prévenir l'inactivation de la β-galactosidase) et décongelés juste avant le traitement par ultra-sons pour détecter la β-galactosidase (Msadek, T., et al 1990. J. Bacteriol. 172:824.834). Les activités spécifiques présentées (exprimées en unités Miller par milligramme de protéine) correspondent aux moyennes d'au moins deux expériences indépendantes.

### - RESULTATS -

### L'expression du gène cryIIIA nécessite la présence d'un fragment d'ADN en amont du gène.

Arantes et Lereclus (1991. Gène 108:115-119) ont montré que le gène cryIIIA était seulement faiblement exprimé dans la souche B. thuringiensis HD1 Cry⁻B lorsqu'il était clone dans un vecteur à faible nombre de copies tel que pHT304 (4 copies par équivalent chromosome).

A partir d'un fragment BamHI de 6 kb portant le gène cryIIIA et les régions adjacentes (figure 1 B), isolé à partir de la souche B. thuringiensis LM79 spécifique pour les coléoptères, on a recherché si des régions en amont du gène pouvaient être impliquées dans la régulation de l'expression de ce gène. Le fragment de 6 kb a été clone dans le site unique BamHI du vecteur pHT304 (figure 1 A) ; des fragments obtenus après digestions partielles ou totales par HindIII du fragment BamHI de 6 kb ont été également insérés de façon indépendante dans le même plasmide pour donner les dérivés pHT305A, pHT305G, pHT305H, pHT305P et pHT305C (figure 1 B). Les cinq plasmides recombinants ont été ensuite introduits dans B thuringiensis sous espèce Kurstaki HD1 Cry⁻ B par électroporation, et les transformants ont été cultivés pendant deux jours à 30°C dans un milieu HCT (Lecadet, M. M., et al 1980. J. Gen. Microbiol. 121:203-212) contenant 25 *µ*g d'érythromicine par ml.

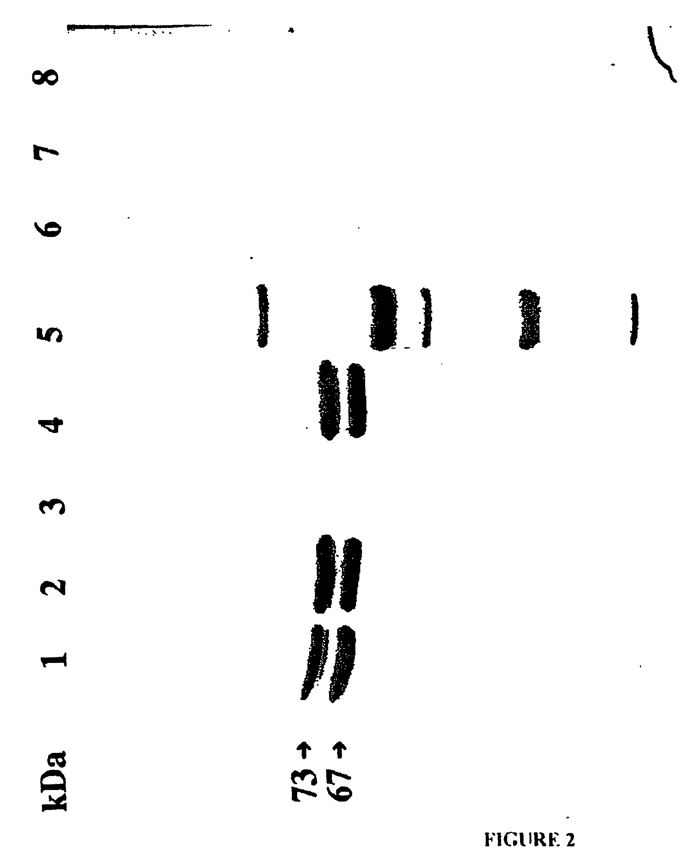

Des préparations de spores comportant des cristaux ont été récupérées à partir des cultures et examinées par microscopie en contraste de phases et SDS-PAGE (figure 2). Les souches recombinantes portant les vecteurs pHT305A, pHT305G et pHT305P (figure 2 lignes 1, 2 et 4 respectivement) produisaient des grandes quantités d'un cristal rhomboïde plat caractéristique des souches actives contre les larves de coléoptères. Les composants principaux de ces cristaux étaient deux peptides d'environ 73 et 67 kDa, tels que ceux préalablement décrits pour les souches de B. thuringiensis portant cryIIIA (Donovan, W.P., et al 1988. Mol. Gen. Genet. 214:365-372).

En revanche, aucune production de cristal n'a été détectée avec les souches portant pHT305H ou pHT305C (figure 2, lignes 3 et 7 respectivement). On a émis l'hypothèse que ces plasmides sont dépourvus de certains éléments présents au contraire dans les dérivés pHT305A, pHT305G et pHT305P. Cet élément supplémentaire éventuel est situé sur un fragment d'ADN de 1 kb, entre le second et le troisième site HindIII, ce fragment étant désigné par H₂-H₃ (figure 1 B). Pour tester si son effet d'activation dépendait de sa position, le fragment H₂-H₃ a été ligaturé à un site SmaI de pHT305H. Dans le plasmide résultant (pHT305HΩ H₂H₃), le fragment H₂H₃ est localisé en aval du gène cryIIIA. La synthèse de la toxine CryIIIA de pHT305HΩH₂H₃ s'est avérée aussi faible qu'avec le plasmide pHT305H (figure 2 ligne 6). Cette absence d'effet pourrait être due soit à la nouvelle localisation du fragment H₂-H₃, cette localisation étant inappropriée ou à la désorganisation de sa structure fonctionnelle. Dans ce cas, l'élement fonctionnnel démarrant à l'intérieur du fragment H₂-H₃ serait étendu à une région au-delà du site HindIII décrit et comprendrait potentiellement la région du promoteur.

### Séquençage de l'ADN et analyse

La séquence nucléotide du fragment H₂-H₃ de 979pb du plasmide pHT791 a été déterminée (figure 3 B). De plus la séquence de 713pb s'étendant du troisième site HindIII au premier site PstI (fragment H₃-P₁) a été déterminée (figure 3 B). Ce second fragment porte la région en amont du promoteur, le promoteur lui-même, le site potentiel de liaison au ribosome, et les premiers 151 codons du gène cryIIIA (Sekar, V. et al, 1987 PNAS. USA 84:7036-7040). Il n'y a pas de différence entre la séquence du fragment H₃-P₁ isolé à partir de la souche LM79 et les régions correspondantes des gènes cryIIIA isolés à partir de B. thuringiensis sous espèce tenebrionis, B. thuringiensis sous espèce san diego et la souche EG2158 (Donovan W.P. et al, Herrnstadt C. et al, Höfte H.J. et al, Sekar V. et al). Aucune séquence potentiellement codante pour une protéine autre que celle correspondant à l'extrémité 5' de cryIIIA n'a été trouvée. Cette région présente une forte proportion de bases A+T (adenine-plus-thymine) correspondant à environ 81 % entre les bases 770 et 990 et deux séquences inversées répétées. La première séquence inversée répétée est imparfaite (16 des 17 pb sont identiques) avec un centre de symétrie au nucléotide 858 et la seconde est une répétition inversée parfaite de 12 pb avec un centre de symétrie au nucléotide 1379. Les énergies libres conduisant à la formation des structures en tige boucle, calculées selon la méthode de Tinoco et al (Tinoco, J.J. et al 1973. Nature (London) New Biol. 246:40-41), étaient respectivement de - 57,7 et - 66,1 kJ/mol.

### Analyse du point d'initiation et de la durée de la transcription en présence du fragment H₂-H₃.

Sekar et al ont cartographié le site de début de la transcription du gène cryIIIA à partir des ARNs isolés à partir de cellules en phase précoce (stade II) et en phase intermédiaire (stades III à IV) de sporulation) en utilisant la nucléase de haricot (mung bean). Ces périodes de croissance correspondent à t₂ à t₅. L'extension à partir d'amorces a été réalisée sur des ARN extraits de cellules en culture à t₀, t₃, t₆ et t₉ pour déterminer si d'autres points d'initiation interviennent lors des phases précoces et tardives de la croissance, et pour déterminer à quel stade la transcription maximale apparaît. Un site de départ de la transcription est apparu sous forme d'un signal faiblement radioactif dans les échantillons à t₀ et t₉ et ce signal s'est avéré plus intense dans les échantillons à t₃ et t₆. Ce site d'initiation de transcription a été cartographié un nucléotide en amont de celui décrit par Sekar et al.

Ces résultats indiquent que le transcrit majoritaire a pour extrémité 5' le T localisé en position 1413 (Fig.3). Cependant, le T localisé en position 1413 (Fig.3) pourrait constituer l'extrémité d'un messager stable dont le vrai démarrage se situerait en amont.

### Détection et analyse quantitative de l'ARNm spécifique de la toxine cryIIIA en présence du fragment H₂-H₃

La transcriptase inverse est satisfaisante pour l'extension à partir d'amorces pour des fragments contenant seulement 100 à 150 bases dans la mesure où cette enzyme peut s'arrêter ou s'interrompre dans des régions comportant d'importantes structures secondaires au niveau de la matrice d'ARN. Pour étudier la présence d'un site potentiel d'initiation de transcription localisé très en amont de l'extrémité 5' du gène cryIIIA, une analyse Northern blot a été réalisée. L'ARN total de la souche portant pHT305P a été récupéré à t₀, t₃, t₆ et t₉. Les ARN ont été séparés par électrophorèse sur gels d'agarose et hybridés avec une sonde correspondant aux fragments internes de cry IIIA marqués (fragment PstI-EcoRI de 874 pb). Dans tous les échantillons, un transcrit principal d'environ 2,5 kb a été détecté. Ceci est cohérent avec la taille du transcrit délimité par le site d'initiation de transcription décrit ci-dessus et une séquence de terminaison potentielle localisée environ 400 pb en aval du codon stop de cryIIIA, décrite par Donovan et al.

Les quantités relatives d'ARNm spécifiques de la toxine CryIIIA synthétisée par la souche portant pHT305P et par la souche portant pHT305H ont été comparées par une procédure dot blot. Des ARN isolés à partir de cultures synchrones récupérées à t₃ ont été immobilisés sur une membrane de nitrocellulose et hybridés à un excès de sonde PstI-EcoRI de cryIIIA. La souche portant pHT305P contenait environ 10 à 15 fois plus d'ARNm spécifique de cryIIIA que la souche contenant pHT305H.

### Production de β-galactosidase à partir de la fusion H₂-H₃ :: lacZ

La synthèse relative du transcrit cryIIIA en présence et en l'absence du fragment H₂-H₃ a indiqué que ce segment d'ADN régule l'expression du gène cryIIIA au niveau de la transcription plutôt qu'au niveau de la traduction. Une fusion avec le gène lacZ a été réalisée pour tester l'effet sur la transcription, produit par le fragment H₂-H₃. Le gène lacZ dépourvu de promoteur a été sous-cloné dans le site SmaI de pHT304. Le plasmide résultant pHT304'lacZ constitue un système permettant de générer des transcriptions de fusion et d'étudier leur expression dans B. thuringiensis dans des conditions approchant celles ayant lieu naturellement avec les gènes cry (plasmide à faible nombre de copies). En conséquence, le fragment H₃-P₁ de 713pb a été cloné entre les sites HindIII et PstI de pHT304'lacZ pour donner pHT7901'lacZ. Finalement, le fragment H₂-H₃ a été cloné dans le site HindIII de pHT7901'lacZ pour donner pHT7902' lacZ, qui porte le fragment H₂-H₃ dans son orientation d'origine par rapport au fragment H₃-P₁ (figure 4). Les plasmides pHT304'lacZ, pHT7901'lacZ et pHT7902'lacZ ont été introduits dans B. thuringiensis sous espèce Kurstaki HD1 Cry⁻B par électroporation. Le vecteur pHT304'lacZ avait un phénotype bleu potentiellement attribué au promoteur lacZ ou à une autre région d'ADN de pUC19 agissant comme promoteur, localisée en amont des sites de clonage. La sporulation de chaque souche a été induite et des échantillons ont été pris à t₋₂ et t₋₁, (2 heures et 1 heure avant le déclenchement de la sporulation, respectivement) et de t₀ à t_{7,5} à 1,5 heure d'intervalle et testés pour l'activité β-galactosidase (figure 5). L'-activité β-galactosidase de la souche portant pHT304'lacZ était constante à environ 800 unités Miller de t₋₂ à t_{7,5}. Le niveau de la production d'enzymes de la souche portant pHT7901'lacZ a monté d'environ 250 unités Miller à t₋₂ jusqu'à environ 1.200 unités Miller à t_{7,5}, indiquant une petite mais significative augmentation de l'activité β-galactosidase pendant la sporulation (cette augmentation n'est pas apparente à cause de l'échelle utilisée sur la figure 5). Au contraire, la souche recombinante portant pHT7902'lacZ a produit beaucoup de β-galactosidase (33.000 unités Miller à t₆ et t_{7,5}). Son activité β-galactosidase a augmenté d'environ 20 fois entre t₀ et t₆ (Figure 5). Le ratio des activités des souches portant pHT7901'lacZ et pHT7902'lacZ a augmenté de 8 fois pendant la phase de croissance végétative à environ 25 fois pendant la phase tardive de la sporulation.

Les résultats présentés ci-dessus et plus précisément les Figures 4 et 5, indiquent que le système d'expression de cryIIIA est fonctionnel (à bas nombre de copies) si la région H₂-H₃ est présente en amont de la région H₃-H₁. Si c'est le cas, de très hauts niveaux d'expression sont obtenus, que ce soit avec le gène cryIIIA ou avec le gène lac Z.

### 1) Définition précise de la région activatrice.

Des délétions du fragment H₂-P₁ (Fig.3 A) ont montré qu'un fragment TaqI - TaqI (positions 907 à 1559, Fig.3) était suffisant pour obtenir la forte expression du gène lac Z (plasmide pHT7930'lacZ, fig.6).

De plus, une délétion interne du fragment, entre les sites PacI et XmnI (positions 990 à 1179) ne réduit pas l'expression du gène lac Z.

Cette délétion interne a fait intervenir un adaptateur entre les sites Pacl et XmnI.

Les deux oligonucléotides suivants ont été synthétisés et hybrides ensemble pour construire une séquence d'ADN double brin pouvant servir d'adaptateur entre les sites PacI et XmnI :

-5'- TAAAGATATCTTTGAAGCTTCACGTGTTTAAACAGGCCTGCAG -3'-
-3'- TAATTTCTATAGAAACTTCGAAGTGCACAAATTTGTCCGGACGTC -5'-

L'adaptateur utilisé ici a une séquence telle que cinq nucléotides naturellement présents après le site PacI, sont reconstitués dans le plasmide pHT7931'lacZ.

En présence de cette délétion, une meilleure expression semble être obtenue en rapprochant les deux régions TaqI - PacI (positions 907 à 990) et XmnI - TaqI (positions 1179 à 1559) (plasmide pHT7931'lacZ, fig.6).

Il s'ensuit que le système d'expression de cryIIIA nécessite l'association de deux séquences d'ADN distinctes ; l'une est comprise entre les sites TaqI et PacI (positions 907 à 990), l'autre est comprise entre les sites XmnI et TaqI(positions 1179 à 1559).

Cette conclusion est renforcée par le fait qu'en l'absence de la région XmnI - TaqI (positions 1179 à 1559), la région située en amont du site XmnI n'est pas suffisante pour obtenir le haut niveau d'expression du gène lac Z (plasmide pHT7907'lacZ, fig.6). En effet, la séquence d'ADN DraI - XmnI (positions 806 à 1179) placée en amont du gène lac Z (plasmide pHT7907'lacZ) ne permet d'obtenir chez Bt (B. thuringiensis) qu'une activité β-galactosidase d'environ 3500 unités Miller (à comparer aux 30.000 u M obtenues avec le plasmide pHT7902'lac Z et pHT7903'lacZ).

Ce résultat confirme donc que l'association des deux séquences TaqI - PacI (positions 907 à 990) et XmnI - TaqI (positions 1179 à 1559) est nécessaire pour que le système d'expression de cryIIIA fonctionne pleinement.

L'expérience réalisée avec le fragment DraI - XmnI en amont de lacZ (plasmide pHT7907'lacZ) indique qu'une activité promotrice est incluse entre DraI et XmnI voire entre TaqI et PacI (positions 907 à 990) puisqu'une forte activité β-galactosidase est obtenue quand le fragment PacI-XmnI (positions 991 à 1179) est absent.

L'analyse des ARN par extension d'amorce réalisée en utilisant un oligonucléotide complémentaire de la séquence comprise entre les positions 1090 et 1121, permet en effet de détecter un démarrage de transcription dans cette région. Celui-ci est localisé en position 983 (Fig.3) ou plus probablement en position 984. Il en résulte qu'un promoteur doit être situé quelques paires de bases en amont de ce démarrage. Bien qu'il n'y ait pas d'homologie évidente avec des promoteurs connus, les boites -35 (TTGCAA) et -10 (TAAGCT) du promoteur se trouveraient entre les positions 945 à 980.

Un fragment d'ADN MunI - PstI (positions 952 à 1612) placé devant lacZ (plasmide pHT7909'lacZ) confère une faible activité β-galactosidase, comparable à celle obtenue avec le plasmide pHT7901'lacZ (Fig. 4 et 5).

Ce résultat suggère que le promoteur situé en positions 945 et 980 peut être inactivé dans une construction débutant à MunI (position 952). On sait toutefois que la séquence minimale nécessaire à l'expression a été définie comme débutant au site TaqI (position 907).

Il résulte de ces différentes expériences, qu'une séquence d'ADN localisée entre les sites TaqI et PacI (positions 907 à 990) est requise pour obtenir une forte expression de lacZ et en conséquence une forte transcription de cryIIIA.

### Mesure de l'activité du promoteur amont dans le système d'expression de cryIIIA

Pour mesurer l'activité du promoteur amont, une fusion transcriptionnelle a été construite avec le fragment d'ADN contenant ce promoteur et le gène lacZ. Pour cela le vecteur d'expression pHT304-18Z a tout d'abord été construit (Fig. 8A). Le fragment d'ADN compris entre les positions 907 et 990 a ensuite été cloné en amont du gène lacZ pour donner le plasmide pHT7832'lacZ. L'activité β-galactosidase est de 3.000 U/mg de protéines à t₀ et de 13.000 U/mg de protéines à t₆ (Fig. 8B).

Le rôle du promoteur amont dans l'activité globale du système d'expression de cryIIIA a été évalué en analysant l'effet produit par son inactivation. Le site de restriction MunI a été rempli à l'aide du fragment Klenow de l'ADN polymérase en présence de deoxynucléotides pour donner le plasmide pHT7832ΔMunI'lacZ. Cela conduit à additionner 4 nucléotides entre les régions -35 et -10 du promoteur (CAATTAATTG versus CAATTG). L'activité β-galactosidase de la souche portant pHT7832Δ MunI'lacZ était d'environ 10 U/mg de protéines à t₀ et d'environ 30 U/mg de protéines à t₆ (Fig. 8B). Ce résultat indique que le promoteur amont est alors inactivé. Le fragment d'ADN contenant le site MunI modifié a été introduit dans le plasmide pHT7830' lacZ pour donner le plasmide pHT7830ΔMunI'lacZ. L'activité β-galactosidase de la souche portant pHT7830ΔMunI' lacZ était d'environ 25 U/mg de protéines à t₀ et d'environ 450 U/mg de protéines à t₆ (Fig. 8B). Par comparaison avec la souche portant le plasmide pHT7830' lacZ, il résulte que le promoteur amont est nécessaire au fonctionnement optimal du système d'expression de cryIIIA. Le plasmide pHT7830' lacZ correspond au vecteur pHT304-18Z dans lequel est clone le fragment TaqI contenant le système d'expression entier de cryIIIA.

### Etude du rôle de la région en aval dans le système d'expression de cryIIIA.

Les résultats précédents confirment que le promoteur amont est nécessaire au fonctionnement optimal du système d'expression de cryIIIA ; en revanche, il n'est pas suffisant pour rendre compte de l'activité maximale du système entier. Ce dernier aspect avait été évoqué précédemment (comparer l'activité β-galactosidase des souches portant les plasmides pHT7832'lacZ et pHT7831'lacZ (Fig. 8B). Le plasmide pHT7831'lacZ correspond au plasmide pHT7830'lacZ dont le fragment interne PacI-XmnI est délété. Il résulte qu'une région dite "aval" est requise pour expliquer l'activité maximale du système d'expression de cryIIIA.

Le site de démarrage de transcription du gène cryIIIA avait été précédemment localisé en position 1413, les régions -35 et -10 du promoteur putatif devaient donc être comprises entre les nucléotides 1370 et 1412 (Sekar et al., 1987, Proc. Natl. Acad. Sci. USA, 84 : 7036-7040). De façon à évaluer l'efficacité de ce promoteur putatif, nous avons construit le plasmide pHT7815/8'lacZ dans lequel le fragment d'ADN compris entre les nucléotides 1352 et 1412 a été délété. L'activité β-galactosidase de la souche portant pHT7815/8'lacZ était d'environ 3.000 U/mg de protéines à t₀ et d'environ 42.000 U/mg de protéines à t₆ (Fig. 8B). Ce résultat indique que la région comprise entre les nucléotides 1362 et 1412 ne joue aucun rôle essentiel dans le système d'expression de cryIIIA et ne peut donc être considérée comme le promoteur du gène cryIIIA.

Une expérience d'extension d'amorce a été réalisée avec les ARN totaux extraits à t₃ d'une souche de B.thuringiensis portant le plasmide pHT7815/8'lacZ. L'extrémité 5' du transcrit majoritaire est détectée, comme précédemment, en position 1413 (Fig. 9). L'ensemble de nos résultats démontrent donc que cette extrémité ne correspond pas à un démarrage de transcription mais à l'extrémité d'un transcrit stable initié en position 984 à partir d'un promoteur amont localisé dans la région d'ADN comprise entre les sites TaqI et PacI (positions 907 à 990) et défini par les régions -35 et -10 : TTGCAA et TAAGCT. L'extrémité 5' du transcrit majoritaire de cryIIIA étant invariablement en position 1413, en présence :ou en absence du fragment d'ADN compris entre les positions 1362 et 1412, il résulte que cette extrémité est déterminée par la présence d'une séquence d'ADN qui se trouve en aval de la position 1413. Le rôle de cette région s'exerce donc à un niveau post-transcriptionnel. L'analyse de cette séquence aval a été faite chez B.subtilis à l'aide de fusions transcriptionnelles avec le gène lacZ. Les différentes constructions présentées figure 10 nous ont permis de délimiter plus précisément la région aval et de mesurer son effet post-transcriptionnel:
1. Le fragment d'ADN compris entre les nucléotides 1462 et 1556 a été délété du plasmide pHT7830'lacZ pour donner le plasmide pHT7816'lacZ. L'activité β-galactosidase de la souche portant pHT7816'lacZ. était d'environ 25.000 U/mg de protéines à t₃, alors que l'activité β-galactosidase de la souche portant pHT7830'lacZ. était d'environ 50.000 U/mg de protéines à t₃ (Fig. 10).
2. Le fragment d'ADN compris entre les nucléotides 1413 et 1556 a été délété du plasmide pHT7830'lacZ pour donner le plasmide pHT7805'lacZ. L'activité β-galactosidase de la souche portant pHT7805'lacZ. était d'environ 5.000 U/mg de protéines à t₃ (Fig. 10).
3. Les nucléotides GGA en position 1421-1423 ont été remplacés, dans le plasmide pHT7830'lacZ, par les nucléotides CCC pour donner le plasmide pHT7830Rm'lacZ. L'activité β-galactosidase de la souche portant pHT7830Rm'lacZ, était d'environ 5.000 U/mg de protéines à t₃ (Fig. 10).
4. Une expérience d'extension d'amorce a été réalisée avec les ARN totaux extraits à t₃ d'une souche de B.thuringiensis portant le plasmide pHT7830Rm'lacZ. L'extrémité 5' du transcrit majoritaire est détectée en position 984 et aucun transcrit ayant une extrémité 5' en position 1413 n'est détecté.

Ces quatre résultats indiquent que l'effet post-transcriptionnel de la région aval est principalement dû à la séquence nucléotidique comprise entre les nucléotides 1413 et 1461. De plus, les nucléotides GGA en position 1421-1423 sont importants pour conférer l'effet post-transcriptionnel et ne pourraient être modifiés qu'en considérant le remplacement par une séquence assurant une intensité d'interaction avec l'ARN 16S du ribosome semblable à l'intensité d'interaction mesurée pour les nucléotides GGA. Par exemple, le remplacement des nucléotides GGA par les nucléotides CCC conduit à la disparition complète de l'effet post-transcriptionnel, expliqué par une forte modification de l'intensité d'interaction entre cette portion du segment et l'ARN 16S. La région aval ainsi définie a comme particularité remarquable de comprendre une séquence nucléotidique complémentaire de l'extrémité 3' de l'ARN 16S des ribosomes.

L'effet post-transcriptionnel de cette séquence d'ADN a ensuite été évalué en utilisant un système d'expression hétérologue : la séquence d'ADN suivante **(S1)** : 
5'-AGCTTGAAAGGAGGGATGCCTAAAAACGAAGAACTGCA-3'
     3'-ACTTTCCTCCCTACGGATTTTTGCTTCTTG-5'
a été synthétisée et clonée entre les sites HindIII et PstI du vecteur pHT304' lacZ pour donner le plasmide pHT304ΩRS1'lacZ. Cette séquence d'ADN est donc intercalée entre le promoteur du gène lacZ et la séquence codante du gène lacZ. L'activité β-galactosidase da la souche 168 de B.subtilis portant pHT304ΩRS1'lacZ. était d'environ 4.000 U/mg de protéines à t₃. Il résulte que la séquence décrite ci-dessus augmente d'un facteur 4 l'expression du gène lacZ. Cette augmentation est comparable à l'augmentation due à la région comprise entre les nucléotides 1413 et 1461 qui était d'un facteur 5 (comparer l'activité β-galactosidase des souches de B.subtilis contenant les plasmides pHT7816'lacZ ou pHT7805' lacZ). La région d'ADN suivante est donc suffisante pour conférer l'effet post-transcriptionnel au système d'expression de cryIIIA) :

5'-CTTGAAAGGAGGGATGCCTAAAAACGAAGAAC-3'
3'-GAACTTTCCTCCCTACGGATTTTTGCTTCTTG-5'

Cette séquence possède une région complémentaire de l'extrémité 3' de l'ARN 16S des ribosomes. Toutefois, d'autres éléments caractéristiques de la région aval du système d'expression de cryIIIA et qui peuvent accentuer cet effet, notamment en empêchant le mouvement du ribosome, sont vraisemblablement inclus dans la séquence nucléotidique comprise entre les positions 1462 et 1556. Leur présence semble expliquer la différence d'activité β-galactosidase observée entre la souche de B.subtilis contenant le plasmide pHT7830'lacZ (50.000 U/mg de protéines à t₃) et la souche de B.subtilis contenant le plasmide pHT7816'lacZ (25.000 U/mg de protéines à t₃ ; voir Fig. 10).

Ces résultats semblent donc confirmer que l'effet post-transcriptionnel de la région aval résulte de l'hybridation entre l'ARN ribosomal 16S et la séquence S2 de l'ARN messager de cryIIIA. Il est donc probable que le ribosome ou une partie du ribosome se fixe sur cette région aval de l'ARN et le protège ainsi d'une dégradation exonucléasique initiée en 5'. Tel que mentionné précédemment, cette fixation aurait donc pour effet d'augmenter la stabilité des messagers et ainsi d'augmenter le niveau d'expression d'un gène donné. Ceci explique que l'extrémité 5' des transcrits de cryIIIA soit invariablement en position 1413, quelle que soit l'initiation de la transcription. Ce mécanisme semble aussi confirmé par l'effet positif de la séquence S1 sur un système d'expression hétérologue (plasmide pHT304'ΩRS1lacZ dans la souche 168 de B.subtilis).

### Introduction de la fusion [système d'expression CryIIIA - LacZ] dans le chromosome de Bacillus subtilis.

Le vecteur pAF1 (fig4) non replicatif chez B.subtilis permet d'introduire les fusions avec le gène reporteur LacZ dans le chromosome de B.subtilis au locus amyE. (J. Bact 1990, 172:835 - 844).
Le plasmide pHC1 est obtenu par insertion du fragment HindIII-SacI (2,7 kb) du pHT7901'Lac Z entre les sites HindIII-SacI du pAF1.

Le plasmide pHC2 est obtenu par insertion du fragment HindIII-SacI (3,7 kb) du pHT7902'LacZ entre les sites HindIII et SacI du pAF1.

Les fusions sont introduites dans la souche B.s 168 trpC2 (Anagnostopoulos,C., et Spizizen,J.1961.J Bacteriol 81:741-746) (Bacillus subtilis 168) par transformation ; le phénotype [amy⁻] rend compte de l'intégration par double recombinaison.

### Etude du système d'expression du gène cryIIIA chez B. Subtilis.

Les souches de B.subtilis obtenues après transformation et intégration des plasmides pHC1 et pHC2 sont appelées respectivement :

### Bs 168 [H] et Bs 168 [P]

La construction contenue dans le plasmide pHC2, c'est-à-dire portant le fragment H₂-P₁ en amont de lac Z, a aussi été introduite dans la souche B.subtilis Δ sigE.

On obtient la souche ΔsigE en transformant une souche parentale (Spo⁺) avec un plasmide non réplicatif chez les bactéries gram-positives et portant un gène sigE dont une région interne est délétée. Le gène sigE a été décrit par Stragier et al 1984. Nature 312:376-378.

La souche ΔsigE est transformée avec le plasmide pHC2 et la souche résultante est ΔsigE [P]. Cette souche a été délétée pour le gène codant pour le facteur sigmaE spécifique de la sporulation. Cette souche est donc asporogène (Spo⁻).

De même, la souche Bs 168 [P] a été transformée avec une "cassette Km^{R}" qui interrompt le gène SpoOA. La souche d'où provient le gène spoOA interrompu par une "cassette Km^{R}" est obtenue en transformant une souche parentale (Spo⁺) avec un plasmide non réplicatif chez les bactéries Gram-positives et portant un gène SpoOA (décrit par Ferrari, F.A et al 1985. PNAS USA 82 : 2647-2651) interrompu par un gène de résistance à la kanamycine. L'ADN chromosomique de cette souche a été utilisé pour transformer la souche Bs 168 [P].

Ainsi la souche résultante Spo⁻ a été appelée Bs168 SpoOA [P].

Il apparaît tout d'abord que la production de β-galactosidase obtenue avec la souche de B.subtilis 168 [H] est très faible (<100 uM) en comparaison avec la souche 168 [P] (environ 15.000 uM). Ces résultats sont similaires à ceux obtenus chez Bt.

De plus, un résultat très surprenant a été obtenu : l'expression dans la souche BsΔsigE est identique à l'expression dans la souche Bs 168 sauvage. Ce résultat indique que le gène cryIIIA n'est pas contrôlé par un promoteur spécifique du facteur sigma E comme c'est le cas pour le gène cryIA.

Il est encore plus surprenant que l'expression dans la souche Bs SpoOA [P] soit supérieure à celle obtenue dans la souche Bs 168 [P]. Ce résultat montre que l'expression de cryIIIA est indépendante de la sporulation puisque le gène spoOA intervient au premier stade de la sporulation.

Ces résultats sont très importants pour le développement et les applications du système d'expression de cryIIIA. Ils indiquent en effet que l'on peut envisager la production des toxines insecticides ou de tout autre protéine d'intérêt commercial dans des souches Spo⁻ de B.subtilis ou de B.thuringiensis.

### Analyse de l'expression de la fusion [système d'expression CryIIIA - LacZ = pHC2] chez Bacillus subtilis en fonction du milieu de culture.

En ce qui concerne l'expression de la fusion dans les milieux 1 à 5 respectivement dont la composition est donnée ci-dessous, on peut faire les observations suivantes :
- Il y a expression (bien que faible) durant la phase végétative.

L'expression augmente à l'entrée en phase stationnaire.
- La comparaison des milieux 2 (carence en phosphate) et 5 (carence en acides aminés) montre que le système d'expression CryIIIA est activé par la carence en acides aminés.
- L'expression dans le milieu 4 montre que cette activation nécessite la présence des sels : CaCl₂, MnnCl₂, CAF.
- L'activation est indépendante de la sporulation :

En milieu de sporulation (milieu Sp) 1 l'expression s'arrête à t₂.

Dans le milieu 5 les cellules ne peuvent sporuler (le glucose inhibe la sporulation), et l'activation est maximum.
- Quand la seule source d'azote est NH⁺₄, l'activation est moins importante, l'expression reste cependant importante (milieu 3).

1/ Milieu Sp : milieu de sporulation
   8 g nutrient hoth (Difco) / litre
   1 mM MgSO₄
   13 mM KCl
   10 µM MnCl₂
   1 µM FeSO₄
   1 mM CaCl₂
2/ Milieu de carence en phosphate
   Tampon HEPES pH7;50 mM
   1 mM MgSO₄
   0,5 mM CaCl₂
   10 µM MnCl₂
   4,4 mg/litre citrate ammônioferrique (CAF)
   2 % glucose
   10 mM KCl
   100 mg/litre chaque acide aminé
   50 mg/litre tryptophane
   0,45 mM tampon phosphate pH 7
**3/ Milieu minimum**
   44 mM KH₂PO₄
   60 mM K₂HPO₄
   2,9 mM citrate tri Nà
   15 mM (NH₄)₂SO₄
   2 % glucose -
4/ Milieu carencé en acides aminés sans CaCl₂
   MnCl₂
   CAF
   44 mM KH₂PO₄
   60 mM K₂HPO₄
   2,9 mM citrate tri Na
   2 % glucose
   1 mM MgSO₄
   50 mg/litre tryptophane
   0,5 hydrelysat de Caséine (HC)
5/ idem 4 en ajoutant :
   0,5 mM CaCl₂
   10 µM MnCl₂
   4,4 mg/litre CAF

### Construction d'une souche de B. thuringiensis Sp⁻.

### Clonage du gène spoOA de B.thuringiensis :

L'ADN total de la souche de B.thuringiensis 407 du sérotype 1 a été purifié et digéré par l'enzyme HindIII. Les fragments HindIII ont été ligaturés avec le vecteur pHT304 digéré par HindIII et le mélange de ligature a été utilisé pour transformer la souche 168 de B.subtilis. Les clones transformants ont été sélectionnés pour la résistance à l'érythromycine. Ils ont ensuite été transformés avec l'ADN total de la souche 168 de B.subtilis dont le gène spoOA était interrompu par une "cassette Km^{R}". Les clones transformants devenus résistants à la kanamycine et ayant toujours un phénotype Spo⁺ ont été étudiés. L'un des clones portait un plasmide recombine capable de compenser la mutation spoOA de B.subtilis. Ce plasmide était constitué du vecteur pHT304 et d'un fragment HindIII d'environ 2,4 kb (Fig. 11A).

### Détermination de la séquence nucléotidique du gène spoOA de B.thuringiensis :

La séquence nucléotidique du fragment HindIII a été déterminée et a révélé la présence d'une phase ouverte de lecture de 804 pb susceptible de coder pour une protéine de 264 acides aminés homologue à la protéine SpoOA de B.subtilis. La séquence nucléotidique de 804 pb du gène spoOA de B.thuringiensis souche 407 est représentée Fig. 11B.

### Interruption du gène spoOA de B.thuringiensis

Un fragment d'ADN de 1,5 kb portant un gène aphIII, conférant la résistance à la kanamycine ("cassette Km^{R}) a été inséré entre les deux sites HincII du gène spoOA (Fig. 11). Un fragment de 40 bp compris entre les positions 267 et 307 du gène SpoOA a donc été remplacé par la "cassette Km^{R}". Le fragment d'ADN HindIII d'environ 3,9 kb contenant le gène spoOA interrompu par la "cassette Km^{R}" a été cloné dans le vecteur thermosensible pRN5101 (Villafane et al. 1987, J. Bacteriol. 169:4822-4829). Le plasmide résultant (désigné pHT5120) a été introduit dans la souche de B.thuringiensis 407 Cry⁻ par électroporation. Le gène spoOA de la souche B.thuringiensis 407 cry⁻ a été remplacé par la copie interrompue avec la "cassette Km^{R}" par recombinaison génétique in vivo en utilisant le protocole précédemment décrit (Lereclus et al. 1992), Bio/Technology 10:418-421). La souche de B. thuringiensis résultante (désignée 407-OA::KM^{R}) est résistante à la kanamycine (300 *µ*g/ml) et ne produit aucune spore lorsqu'elle est cultivée en milieu HCT, habituellement favorable à la sporulation de B.thuringiensis. Une expérience d'hybridation ADN/ADN, réalisée avec le fragment HindIII de 2,4 kb comme sonde, révèle que le gène spoOA de la souche B.thuringiensis 407 Cry⁻ a bien été remplacé par la copie interrompue avec la "cassette Km^{R}".

### Production de la toxine CryIIIA dans la souche B.thuringiensis 407-OA::Km^{R}:

Le plasmide pHT305P, portant le gène cryIIIA, a été introduit dans la souche B.thuringiensis 407-OA::Km^{R} par électroporation. Le clone recombinant obtenu a été déposé à la CNCM le 03/05/1994 et pour lequel le numéro d'accession I-1412 lui a été assigné. Le clone recombinant obtenu a été cultivè à 30°C en milieu HCT + glucose 3 g/l ou en milieu LB (NaCl, 5 g/l : extrait de levure, 5 g/l ; Bacto tryptone 10 g/l) pour estimer la production de toxines. Après environ 48 heures les bactéries contenaient un cristal visible par examen en microscopie optique. Ce cristal était de forme rhomboidale, caractéristique des cristaux constitués par la protéine CryIIIA. Les cristaux produits par la souche B.thuringiensis 407-OA::Km^{R} [pHT315] sont de taille importante et restent inclus dans les cellules plusieurs jours après que celles-ci aient cessé de se développer en milieu HCT ; en milieu LB une partie des cellules lysent et les cristaux sont libérés. Les cristaux sont constitués de protéines d'environ 70 kDa (CryIIIA) spécifiquement toxiques pour les coléoptères.

## Revendications

1. Séquence d'ADN de contrôle de l'expression d'une séquence codante d'un gène dans une cellule hôte, **caractérisée en ce qu'**elle est constituée :
a) d'un promoteur et
b) d'une séquence nucléotidique, exerçant un rôle au niveau post-transcriptionnel, constituée d'une partie du fragment d'ADN de 1692 pb environ, délimité par les sites de restriction HindIII et Pst1 (fragment H₂-P₁) représenté à la figure 3A, et comportant une région complémentaire à l'extrémité 3' d'un ARN 16S ribosomal bactérien, ladite séquence exerçant un rôle au niveau post-transcriptionnel étant située en aval et sous le contrôle dudit promoteur.

2. Séquence d'ADN selon la revendication 1, **caractérisée en ce que** l'enchaînement d'ADN de 1692 pb environ, délimité par les sites de restriction HindIII-Pst1 (fragment H₂-P₁), est tel qu'obtenu par digestion partielle du fragment BamHI de 6 kb portant le gène cryIIIA de Bacillus thuringiensis souche LM79, ladite séquence d'ADN étant en outre susceptible d'intervenir dans le contrôle de l'expression d'une séquence codante de nucléotides placée en aval, dans un hôte cellulaire bactérien, particulièrement de type Bacillus thuringiensis et/ou Bacillus subtilis.

3. Séquence d'ADN selon la revendication 1 **caractérisée en ce qu'**elle correspond à l'enchaînement HindIII-Pst1 d'une longueur de 1692 pb environ (fragment H₂-P₁) représenté à la figure 3A.

4. Séquence d'ADN selon la revendication 1, **caractérisée en ce qu'**elle correspond à l'enchaînement de nucléotides suivant, désigné par l'expression Seq N° 1, compris entre les nucléotides 1 et 1692 :

AAGCTTTCAG TGAAGTACGT GATTATACGG AGATGAAAAT TCGTACACTG TTAACGAGAA 60

GGAAACGCCG ACGAAAGCGT AGCATCGGAT GGCAAAGATG GAGTAACGAA TATCTCTACG 120

GTGTACTGGG GCTTTACTGA GACTAGAAAG TCCTTCCCTT GAAAAGTGCA GAGAGTTTTC 180
GATAAAAGTG TCAGCCATTT GATAAGTCTC ATTCTCATAA CCTATTGATG AAGTTTATAG 240
GGAAGCTGCT TGAGAGGGAA AACCTCACGA ACAGTTCTTA TGGGGAGAGA CTGGAAACAG 300
GTCACAATTG ATACCTCGCT AATCTTTTAA CCGACAAAGT TTTTTTAAAC CGTGGAAGTC 360
ATAATAACCT GGATATTGTG AATTTATAAA AGTTAACAAA TGGTTTATAT TAAGACAGTC 420
ATAAACCAAA GATTTTTCTT CTAAAGCTAC GATAGCAAAA ATTTCACTAG AAATTAGTTA 480
TACAAGCATT TTGTAAGAAT TATTAAAAAG ATAAATCCTG CTATTACGAG ATTAGTAGGA 540
TGATATTGTG AAAAATTTTT TATCTATTCG ATTTAAAATA TTTATGAATT TTACATAAAC 600
CTCATAAGAA AAAATACTAT CTATACTATT TTAAGAAATT TATTAGAATA AGCGGATTCA 660
AAATAGCCCT GGCCATAAAA GTACCTCAGC AGTAGAAGTT TTGACCAAAA TTAAAAAAAT 720
ACCCAATCAA GAGAATATTC TTAATTACAA TACGTTTTGC GAGGAACATA TTGATTGAAA 780
TTTAATAAAT TTAGTCCTAA AATTTAAAGA AATTTAAGTT TTTCATATTT TTATGAACTA 840
ACAAGAATAA AAATTGTGTT TATTTATTAT TCTTGTTAAA TATTTGATAA AGAGATATAT 900
TTTTGGTCGA AACGTAAGAT GAAACCTTAG ATAAAAGTGC TTTTTTTGTT GCAATTGAAG 960
AATTATTAAT GTTAAGCTTA ATTAAAGATA ATATCTTTGA ATTGTAACGC CCCTCAAAAG 1020
TAAGAACTAC AAAAAAAGAA TACGTTATAT AGAAATATGT TTGAACCTTC TTCAGATTAC 1080
AAATATATTC GGACGGACTC TACCTCAAAT GCTTATCTAA CTATAGAATG ACATACAAGC 1140
ACAACCTTGA AAATTTGAAA ATATAACTAC CAATGAACTT GTTCATGTGA ATTATCGCTG 1200
TATTTAATTT TCTCAATTCA ATATATAATA TGCCAATACA TTGTTACAAG TAGAAATTAA 1260
GACACCCTTG ATAGCCTTAC TATACCTAAC ATGATGTAGT ATTAAATGAA TATGTAAATA 1320
TATTTATGAT AAGAAGCGAC TTATTTATAA TCATTACATA TTTTTCTATT GGAATGATTA 1380
AGATTCCAAT AGAATAGTGT ATAAATTATT TATCTTGAAA GGAGGGATGC CTAAAAACGA 1440
AGAACATTAA AAACATATAT TTGCACCGTC TAATGGATTT ATGAAAAATC ATTTTATCAG 1500
TTTGAAAATT ATGTATTATG ATAAGAAAGG GAGGAAGAAA AATGAATCCG AACAATCGAA 1560
GTGAACATGA TACAATAAAA ACTACTGAAA ATAATGAGGT GCCAACTAAC CATGTTCAAT 1620
ATCCTTTAGC GGAAACTCCA AATCCAACAC TAGAAGATTT AAATTATAAA GAGTTTTTAA 1680
GAATGACTGC AG 1692

5. Séquence d'ADN selon l'une quelconque des revendications 1 ou 2, **caractérisée en ce qu'**elle correspond à l'enchaînement délimité par les sites de restriction TaqI-TaqI.

6. Séquence d'ADN selon l'une quelconque des revendications 1 ou 2, **caractérisée en ce qu'**elle correspond à l'enchaînement de nucléotides désigné par l'expression Seq N° 2 compris entre les nucléotides 907 et 1559 de la séquence de la figure 3.

7. Séquence d'ADN selon l'une quelconque des revendications 1 ou 2, **caractérisée en ce qu'**elle comprend le fragment TaqI-PacI répondant à l'enchaînement Seq N° 3, en association avec le fragment XmnI-TaqI répondant à l'enchaînement Seq N° 4.

8. Séquence d'ADN selon l'une quelconque des revendications 1 à 7, **caractérisée en ce qu'**elle intervient dans le contrôle de la transcription de la séquence codante de nucléotides et pour augmenter l'expression d'une séquence codante.

9. Séquence d'ADN selon les revendications 1 à 8, **caractérisée par** :
- sa capacité d'hybridation dans des conditions non stringentes avec un enchaînement de nucléotides choisi parmi les enchaînements Seq N° 1, Seq N° 2, Seq N° 3 et Seq N° 4, et par
- sa capacité à intervenir dans le contrôle de l'expression, dans un hôte cellulaire, particulièrement du type Bacillus, d'une séquence codante de nucléotides placée en aval.

10. Séquence selon la revendication 1, **caractérisée en ce que** ladite séquence nucléotidique exerçant un rôle au niveau post-transcriptionnel est la séquence S2 suivante : 
5'-CTTGAAAGGAGGGATGCCTAAAAACGAAGAAC-3'
3'-GAACTTTCCTCCCTACGGATTTTTGCTTCTTG-5'
 isolée d'une bactérie, particulièrement d'une bactérie Gram+ , plus particulièrement d'une bactérie de type Bacillus, ou une séquence dérivée de ladite séquence S2, ladite séquence S2 étant **caractérisée en ce qu'**elle comporte une région essentiellement complémentaire à l'extrémité 3' de l'ARN 16S d'un ribosome bactérien, et **en ce qu'**elle est susceptible d'intervenir au niveau post-transcriptionnel lors de l'expression de ladite séquence codante, notamment sans agir directement sur la traduction de la séquence codante à exprimer, particulièrement lorsque ladite séquence S2 est située entre ledit promoteur et ladite séquence codante d'un gène, afin d'augmenter l'expression de ladite séquence codante chez une bactérie, particulièrement une bactérie de type Bacillus.

11. Séquence selon la revendication 10, **caractérisée en ce qu'**elle comporte une première région suffisamment complémentaire à l'extrémité 3' de l'ARN 16S d'un ribosome bactérien, particulièrement d'une bactérie Gram+, plus particuüèrement d'une bactérie de type Bacillus, pour que ladite première région soit susceptible de s'hybrider à un ribosome bactérien ou à une partie dudit ribosome, et une deuxième région en aval de la première région, ladite deuxième région comportant une séquence susceptible d'avoir un effet positif additionnel au niveau de l'expression de la séquence codante.

12. Séquence d'ADN selon la revendication 1, **caractérisée en ce que** la séquence nucléotidique exerçant un rôle au niveau post-transcriptionnel présente les propriétés suivantes :
- elle est contenue dans un enchaînement de nucléotides hybridant dans des conditions non stringentes avec le fragment d'ADN compris entre les nucléotides 1413 et 1559 de la séquence représentée à la figure 3 ;
- elle est susceptible d'intervenir dans le contrôle de l'expression de la séquence codante d'un gène dans une cellule hôte, notamment d'une séquence codant pour un polypeptide de Bacillus toxique vis-à-vis des insectes ou d'une séquence codant pour un polypeptide exprimé pendant la phase végétative, pendant la phase stationnaire ou pendant la sporulation de Bacillus.

13. Séquence d'ADN selon l'une quelconque des revendications 1 ou 2, **caractérisée en ce que** la séquence nucléotidique exerçant un rôle au niveau post-transcriptionnel correspond à l'enchaînement délimité par les sites de restriction XmnI-TaqI.

14. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 4 compris entre les nucléotides 1179 et 1559 de la séquence de la figure 3, ou **en ce qu'**elle correspond à toute partie d'au moins 10 nucléotides naturellement consécutifs ou non de cet enchaînement, susceptible d'intervenir dans le contrôle de l'expression d'une séquence codante de nucléotides placée en aval dans un hôte cellulaire.

15. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 5 correspondant au fragment comprenant les nucléotides 1179 à 1556 de la séquence représentée à la figure 3.

16. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 11 correspondant au fragment comprenant les nucléotides 1413 à 1556 de la séquence représentée à la figure 3.

17. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 8 correspondant au fragment comprenant les nucléotides 1413 à 1461 de la séquence représentée à la figure 3.

18. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 9 correspondant au fragment d'ADN suivant :

5'-AGCTTGAAAGGAGGGATGCCTAAAAACGAAGAACTGCA-3'
3'-ACTTTCCTCCCTACGGATTTTTGCTTCTTG-5'

19. Séquence d'ADN selon l'une quelconque des revendications 1 ou 2, **caractérisée en ce que** la séquence nucléotidique exerçant un rôle au niveau post-transcriptionnel correspond à l'enchaînement Seq N° 10 correspondant au fragment d'ADN suivant :

5'-CTTGAAAGGAGGGATGCCTAAAAACGAAGAAC-3'
3'-GAACTTTCCTCCCTACGGATTTTTGCTTCTTG-5'

20. Séquence d'ADN selon l'une quelconque des revendications 1 ou 2, **caractérisée en ce que** la séquence nucléotidique exerçant un rôle au niveau post-transcriptionnel correspond au fragment d'ADN suivant :

5'–GAAAGGAGG-3'
3'–CTTTCCTCC-5'

21. Séquence d'ADN selon l'une quelconque des revendications 1 à 20, **caractérisée en ce que** le promoteur est inclus dans l'enchaînement délimité par les sites de restriction TaqI-PacI.

22. Séquence d'ADN selon la revendication 21, **caractérisée en ce que** le promoteur correspond à l'enchaînement Seq N° 3 compris entre les nucléotides 907 et 990 de la séquence de la figure 3, ou un variant comprenant les nucléotides 907 à 985, ou **caractérisée en ce que** le promoteur correspond à toute partie d'au moins 10 nucléotides naturellement consécutifs ou non de cet enchaînement, susceptible d'intervenir dans le contrôle de l'expression d'une séquence codante de nucléotides placée en aval dans un hôte cellulaire bactérien.

23. Séquence nucléotidique S2 de séquence : 
5'-CTTGAAAGGAGGGATGCCTAAAAACGAAGAAC-3'
3'-GAACTTTCCTCCCTACGGATTTTTGCTTCTTG-5'
 comportant une région suffisamment complémentaire à l'extrémité 3' de l'ARN 16S d'un ribosome bactérien, particulièrement d'une bactérie Gram+, plus particulièrement d'une bactérie de type Bacillus pour être susceptible de s'hybrider à un ribosome bactérien ou une partie d'un ribosome bactérien.

24. Séquence nucléotidique **caractérisée en ce qu'**elle est comprise dans l'enchaînement Seq N° 5 correspondant au fragment comprenant les nucléotides 1179 à 1556 de la séquence représentée à la figure 3, et qu'elle comporte une région essentiellement complémentaire à l'extrémité 3' de l'ARN 16S d'un ribosome bactérien, ladite séquence étant susceptible d'intervenir au niveau post-transcriptionnel lors de l'expression de la séquence codante d'un gène, notamment sans agir directement sur la traduction de la séquence codante à exprimer, ladite séquence nucléotidique étant sous le contrôle d'un promoteur contrôlant ladite séquence codante.

25. Séquence nucléotidique selon la revendication 24, **caractérisée en ce que** la séquence nucléotidique correspond à l'enchaînement Seq N° 5 correspondant au fragment comprenant les nucléotides 1179 à 1556 de la séquence représentée à la figure 3.

26. Séquence nucléotidique selon la revendication 24, **caractérisée en ce que** la séquence nucléotidique correspond à l'enchaînement Seq N° 11 correspondant au fragment comprenant les nucléotides 1413 à 1556 de la séquence représentée à la figure 3.

27. Séquence nucléotidique selon la revendication 24, **caractérisée en ce que** la séquence nucléotidique correspond à l'enchaînement Seq N° 8 correspondant au fragment comprenant les nucléotides 1413 à 1461 de la séquence représentée à la figure 3.

28. Séquence nucléotidique selon la revendication 24, **caractérisée en ce que** la séquence nucléotidique exerçant un rôle au niveau post-transcriptionnel correspond à l'enchaînement Seq N° 9 correspondant au fragment d'ADN suivant :

5'-AGCTTGAAAGGAGGGATGCCTAAAAACGAAGAACTGCA-3'
3'-ACTTTCCTCCCTACGGATTTTTGCTTCTTG-5'

29. Séquence nucléotidique selon la revendication 24, **caractérisée en ce que** la séquence nucléotidique correspond au fragment d'ADN suivant :

5'-GAAAGGAGG-3'
3'-CTTTCCTCC-5'

30. Séquence d'ADN recombinée, **caractérisée en ce qu'**elle est constituée par :
a. une séquence codante déterminée,
b. une séquence nuctéotidique constituée par un promoteur et une séquence exerçant un rôle au niveau post-transcriptionnel, selon l'une quelconque des revendications 12 à 20;
ladite séquence exerçant un rôle au niveau post-transcriptionnel étant située en aval dudit promoteur et en amont de ladite séquence codante.

31. Séquence d'ADN recombinée, **caractérisée en ce qu'**elle comprend :
a. une séquence codante déterminée, sous le contrôle d'un promoteur,
b. une séquence nucléotidique exerçant un rôle au niveau post-transcriptionnel selon l'une quelconque des revendications 23 à 29,
ladite séquence exerçant un rôle au niveau post-transcriptionnel étant située en aval dudit promoteur et en aval de ladite séquence codante.

32. Séquence d'ADN recombinée selon la revendication 32, **caractérisée en ce qu'**elle comprend en outre une séquence exerçant un rôle au niveau post-transcriptionnel, située en amont de ladite séquence codante.

33. Séquence d'ADN recombinée selon l'une des revendications 30 à 32, **caractérisée en ce que** la séquence codante de nucléotides qu'elle contient est une séquence codant pour une enzyme.

34. Séquence d'ADN recombinée selon l'une des revendications 30 à 32, **caractérisée en ce que** la séquence codante de nucléotides qu'elle contient est une séquence codant pour un antigène.

35. Séquence d'ADN recombinée selon l'une des revendications 30 à 32, **caractérisée en ce que** la séquence codante de nucléotides qu'elle contient est une séquence toxique vis-à-vis des insectes et en particulier une séquence à activité larvicide.

36. Vecteur d'expression obtenu par insertion d'une séquence d'ADN constituée d'une séquence recombinée selon l'une quelconque des revendications 5 à 32, de façon telle que ladite séquence d'ADN recombinée intervienne dans le contrôle de l'expression d'une séquence codante de nucléotides déterminée.

37. Vecteur d'expression selon la revendication 36, **caractérisé en ce qu'**il s'agit d'un plasmide, par exemple d'un plasmide intégratif ou d'un plasmide réplicatif.

38. Vecteur d'expression selon la revendication 37, **caractérisé en ce qu'**il s'agit du plasmide pHT7902'lacZ déposé à la CNCM le 20 avril 1993 sous le n° l-1301.

39. Hôte cellulaire recombiné, **caractérisé en ce qu'**il est modifié par une séquence d'ADN selon l'une quelconque des revendications 1 à 35 ou par un vecteur d'expression selon l'une quelconque des revendications 36 à 38.

40. Hôte cellulaire selon la revendication 39, **caractérisé en ce qu'**il s'agit d'un Bacillus, par exemple de B. thuringiensis ou de B. subtilis.

41. Hôte cellulaire selon la revendication 40, **caractérisé en ce qu'**il s'agit d'une souche asporogène de Bacillus, par exemple d'une souche de B. subtilis asporogène, en particulier une souche capable d'exprimer la séquence codante de la séquence d'ADN selon l'une quelconque des revendications 14 à 18, pendant la phase stationnaire de croissance.

42. Hôte cellulaire, **caractérisé en ce qu'**il s'agit de la souche 407-OA ::Km^{R} (pHT305D) déposée à la CNCM le 3 mai 1994 sous le n° 1-1412.

43. Procédé de production d'une protéine recombinante codée par une séquence de nucléotides déterminée, ledit procédé étant **caractérisé en ce qu'**il comprend :
- l'introduction dans un hôte cellulaire d'un vecteur selon l'une quelconque des revendications 36 à 38,
- la croissance dudit hôte cellulaire à des conditions permettant l'expression de ladite séquence de nucléotides déterminée, et
- la récupération de la protéine recombinante.

44. Utilisation d'une séquence nucléotidique selon l'une des revendications 1 à 22, pour le contrôle de l'expression de la séquence codante d'un gène.

45. Utilisation d'une séquence nucléotidique selon l'une des revendications 1 à 22, pour l'obtention d'un haut niveau d'expression de la séquence codante d'un gène.

46. Utilisation d'une séquence nucléotidique selon l'une des revendications 23 à 29 pour la régulation post-transcriptionnelle du produit de transcription de la séquence codante d'un gène.

47. Utilisation d'une séquence nucléotidique selon l'une quelconque des revendications 23 à 29 pour augmenter la quantité d'ARNm d'une séquence codante d'un gène.

## Patentansprüche

1. DNA-Sequenz zur Expressionskontrolle einer codierenden Sequenz eines Gens in einer Wirtszelle, **dadurch gekennzeichnet, dass** sie aufgebaut ist:
(a) aus einem Promotor und
(b) aus einer Nucleotidsequenz, welche eine Funktion auf postranskriptioneller Ebene ausübt, zusammengesetzt aus einem Teil des DNA-Fragments aus ungefähr 1692 Basenpaaren, begrenzt durch die Restriktionsschnittstellen HindIII und Pst1 (Fragment H₂-P₁), dargestellt in der Abbildung 3A und enthaltend eine Region, die komplementär ist zum 3'-Ende einer bakteriellen ribosomalen 16S-RNA, wobei die Sequenz; die auf postranskriptioneller Ebene eine Funktion ausübt, sich stromabwärts und unter der Kontrolle des Promotors befindet.

2. DNA-Sequenz nach Anspruch 1, **dadurch gekennzeichnet, dass** die DNA-Sequenz aus ungefähr 1692 Basenpaaren, begrenzt durch die Restriktionsschnittstellen HindIII-Pst1 (Fragment H₂-P₁), dergestalt ist, wie die durch partiellen Verdau des 6 kb-BamHI-Fragments erhaltene, welche das Gen cryIIIA von Bacillus thuringiensis des Stammes LM79 enthält, wobei die DNA-Sequenz desweiteren die Expressionskontrolle einer codierenden Nucleotidsequenz, welche stromabwärts platziert ist, in einer bakteriellen Wirtszelle beeinflussen kann, insbesondere des Typs Bacillus thuringiensis und/oder Bacillus subtilis.

3. DNA-Sequenz nach Anspruch 1, **dadurch gekennzeichnet, dass** sie der in der Abbildung 3A dargestellten Sequenz HindIII-Pst1 mit einer Länge von ungefähr 1692 Basenpaaren (Fragment H₂-P₁) entspricht.

4. DNA-Sequenz nach Anspruch 1, **dadurch gekennzeichnet, dass** sie der folgenden Nucleotidsequenz entspricht, dargestellt durch Seq. Nr. 1, welche von den Nucleotiden 1 bis 1692 umfasst ist:

AAGCTTTCAG TGAAGTACGT GATTATACGG AGATGAAAAT TCGTACACTG TTAACGAGAA 60
GGAAACGCCG ACGAAAGCGT AGCATCGGAT GGCAAAGATG GAGTAACGAA TATCTCTACG 120
GTGTACTGGG GCTTTACTGA GACTAGAAAG TCCTTCCCTT GAAAAGTGCA GAGAGTTTTC 180
GATAAAAGTG TCAGCCATTT GATAAGTCTC ATTCTCATAA CCTATTGATG AAGTTTATAG 240
GGAAGCTGCT TGAGAGGGAA AACCTCACGA ACAGTTCTTA TGGGGAGAGA CTGGAAACAG 300
GTCACAATTG ATACCTCGCT AATCTTTTAA CCGACAAAGT TTTTTTAAAC CGTGGAAGTC 360
ATAATAACCT GGATATTGTG AATTTATAAA AGTTAACAAA TGGTTTATAT TAAGACAGTC 420
ATAAACCAAA GATTTTTCTT CTAAAGCTAC GATAGCAAAA ATTTCACTAG AAATTAGTTA 480
TACAAGCATT TTGTAAGAAT TATTAAAAAG ATAAATCCTG CTATTACGAG ATTAGTAGGA 540
TGATATTGTG AAAAATTTTT TATCTATTCG ATTTAAAATA TTTATGAATT TTACATAAAC 600
CTCATAAGAA AAAATACTAT CTATACTATT TTAAGAAATT TATTAGAATA AGCGGATTCA 660
AAATAGCCCT GGCCATAAAA GTACCTCAGC AGTAGAAGTT TTGACCAAAA TTAAAAAAAT 720
ACCCAATCAA GAGAATATTC TTAATTACAA TACGTTTTGC GAGGAACATA TTGATTGAAA 780
TTTAATAAAT TTAGTCCTAA AATTTAAAGA AATTTAAGTT TTTCATATTT TTATGAACTA 840
ACAAGAATAA AAATTGTGTT TATTTATTAT TCTTGTTAAA TATTTGATAA AGAGATATAT 900
TTTTGGTCGA AACGTAAGAT GAAACCTTAG ATAAAAGTGC TTTTTTTGTT GCAATTGAAG 960
AATTATTAAT GTTAAGCTTA ATTAAAGATA ATATCTTTGA ATTGTAACGC CCCTCAAAAG 1020
TAAGAACTAC AAAAAAAGAA TACGTTATAT AGAAATATGT TTGAACCTTC TTCAGATTAC 1080
AAATATATTC GGACGGACTC TACCTCAAAT GCTTATCTAA CTATAGAATG ACATACAAGC 1140
ACAACCTTGA AAATTTGAAA ATATAACTAC CAATGAACTT GTTCATGTGA ATTATCGCTG 1200
TATTTAATTT TCTCAATTCA ATATATAATA TGCCAATACA TTGTTACAAG TAGAAATTAA 1260
GACACCCTTG ATAGCCTTAC TATACCTAAC ATGATGTAGT ATTAAATGAA TATGTAAATA 1320
TATTTATGAT AAGAAGCGAC TTATTTATAA TCATTACATA TTTTTCTATT GGAATGATTA 1380
AGATTCCAAT AGAATAGTGT ATAAATTATT TATCTTGAAA GGAGGGATGC CTAAAAACGA 1440
AGAACATTAA AAACATATAT TTGCACCGTC TAATGGATTT ATGAAAAATC ATTTTATCAG 1500
TTTGAAAATT ATGTATTATG ATAAGAAAGG GAGGAAGAAA AATGAATCCG AACAATCGAA 1560
GTGAACATGA TACAATAAAA ACTACTGAAA ATAATGAGGT GCCAACTAAC CATGTTCAAT 1620
ATCCTTTAGC GGAAACTCCA AATCCAACAC TAGAAGATTT AAATTATAAA GAGTTTTTAA 1680
GAATGACTGC AG 1692

5. DNA-Sequenz nach einem der Ansprüche 1 oder 2, **dadurch gekennzeichnet, dass** sie der Sequenz entspricht, welche durch die Restriktionsschnittstellen TaqI-TaqI begrenzt ist.

6. DNA-Sequenz nach einem der Ansprüche 1 oder 2, **dadurch gekennzeichnet, dass** sie der Nucleotidsequenz entspricht, dargestellt durch Seq. Nr. 2, welche von den Nukleotiden 907 bis 1559 der Sequenz aus der Abbildung 3 umfasst ist.

7. DNA-Sequenz nach einem der Ansprüche 1 oder 2, **dadurch gekennzeichnet, dass** sie das TaqI-PacI Fragment, das der Seq. Nr. 3 entspricht, zusammen mit dem XmnI-TaqI Fragment, das der Seq. Nr. 4 entspricht, umfasst.

8. DNA-Sequenz nach einem der Ansprüche 1 bis 7, **dadurch gekennzeichnet, dass** sie in die Transkriptionskontrolle der codierenden Nucleotidsequenz eingreift, um die Expression einer codierenden Sequenz zu erhöhen.

9. DNA-Sequenz nach den Ansprüchen 1 bis 8, **gekennzeichnet durch**:
- ihre Fähigkeit zur Hybridisierung unter nicht-stringenten Bedingungen mit einer Nucleotidsequenz ausgewählt aus den Sequenzen SEQ ID Nr. 1, SEQ ID Nr. 2, SEQ ID Nr. 3 und SEQ ID Nr. 4, und **durch**
- ihre Fähigkeit, die Expressionskontrolle einer codierenden Nucleotidsequenz, welche stromabwärts platziert ist, in einer Wirtszelle, vorzugsweise des Typs Bacillus zu beeinflussen.

10. Sequenz nach Anspruch 1, **dadurch gekennzeichnet, dass** die Nucleotidsequenz, welche eine Funktion auf postranskriptioneller Ebene ausübt, die folgende Sequenz S2 ist: 
5'-CTTGAAAGGAGGGATGCCTAAAAACGAAGAAC-3'
3'-GAACTTTCCTCCCTACGGATTTTTGCTTCTTG-5'
isoliert aus einem Bakterium, vorzugsweise aus einem Gram+-Bakterium, mehr bevorzugt aus einem Bakterium des Typs Bacillus, oder eine Sequenz abgeleitet aus der Sequenz S2, wobei die Sequenz S2 **dadurch gekennzeichnet ist, dass** sie eine Region enthält, welche im Wesentlichen komplementär zum 3'-Ende der bakteriellen ribosomalen 16S-RNA ist, und **dadurch**, dass sie in der Lage ist, auf posttranskripitioneller Ebene während der Expression der codierenden Sequenz einzugreifen, insbesondere ohne direkt auf die Translation der zu exprimierenden codierenden Sequenz einzuwirken, vorzugsweise, wenn sich die Sequenz S2 zwischen dem Promotor und der codierenden Sequenz eines Gens befindet, um die Expression der codierenden Sequenz in einem Bakterium, vorzugsweise einem Bakterium des Typs Bacillus, zu erhöhen.

11. Sequenz nach Anspruch 10, **dadurch gekennzeichnet, dass** sie eine erste Region enthält, welche ausreichend komplementär zum 3'-Ende der bakteriellen ribosomalen 16S-RNA ist, vorzugsweise eines Gram+ Bakteriums, mehr bevorzugt eines Bakteriums des Typs Bacillus, so dass die erste Region in der Lage sein kann, an ein bakterielles Ribosom zu hybridisieren oder an einen Teil des Ribosoms, und eine zweite Region stromabwärts von der ersten Region, wobei die zweite Region eine Sequenz enthält, welche in der Lage ist, einen zusätzlichen positiven Effekt auf das Expressionsniveau der codierenden Sequenz zu haben.

12. DNA-Sequenz nach Anspruch 1, **dadurch gekennzeichnet, dass** die Nucleotidsequenz, welche eine Funktion auf posttranskriptioneller Ebene ausübt, die folgenden Eigenschaften aufweist:
- sie ist enthalten in einer Nucleotidsequenz, welche unter nicht-stringenten Bedingungen mit dem DNA-Fragment hybridisiert, welches von den Nucleotiden 1413 bis 1559 der in Abbildung 3 dargestellten Sequenz umfasst ist;
- sie ist in der Lage, die Expressionskontrolle der codierenden Sequenz eines Gens in einer Wirtszelle zu beeinflussen, insbesondere einer Sequenz, die ein Polypeptid von Bacillus codiert, welches toxisch gegenüber Insekten ist, oder einer Sequenz, die ein Polypeptid codiert, das während der vegetativen Phase, während der stationären Phase oder während der Sporulation von Bacillus exprimiert wird.

13. DNA-Sequenz nach einem der Ansprüche 1 oder 2, **dadurch gekennzeichnet, dass** die Nucleotidsequenz, welche eine Funktion auf posttranskriptioneller Ebene ausübt, der Sequenz entspricht, welche durch die Restriktionsschnittstellen XmnI-TaqI begrenzt ist.

14. DNA-Sequenz nach einem der Ansprüche 1 oder 2, **dadurch gekennzeichnet, dass** die Nucleotidsequenz, welche eine. Funktion auf posttranskriptioneller Ebene ausübt, der Sequenz Nr. 4 entspricht, welche von den Nucleotiden 1179 bis 1559 der Sequenz aus Abbildung 3 umfasst ist, oder **dadurch gekennzeichnet, dass** sie in jedem Teil mindestens 10 natürlicherweise aufeinanderfolgenden oder nicht natürlicherweise aufeinanderfolgenden Nucleotiden dieser Sequenz entspricht und in der Lage ist, in die Expressionskontrolle einer codierenden Nucleotidsequenz, welche stromabwärts platziert ist, in einer Wirtszelle einzugreifen.

15. DNA-Sequenz nach einem der Ansprüche 1 oder 2, **dadurch gekennzeichnet dass** die Nucleotidsequenz, welche eine Funktion auf posttranskriptioneller Ebene ausübt, der Sequenz Nr. 5 entspricht, welche dem Fragment entspricht, das die Nucleotide 1179 bis 1556 der in Abbildung 3 dargestellten Sequenz umfasst.

16. DNA-Sequenz nach einem der Ansprüche 1 oder 2, **dadurch gekennzeichnet dass** die Nucleotidsequenz, welche eine Funktion auf posttranskriptioneller Ebene ausübt, der Sequenz Nr. 11 entspricht, welche dem Fragment entspricht, das die Nucleotide 1413 bis 1556 der in Abbildung 3 dargestellten Sequenz umfasst.

17. DNA-Sequenz nach einem der Ansprüche 1 oder 2, **dadurch gekennzeichnet dass** die Nucleotidsequenz, welche eine Funktion auf posttranskriptioneller Ebene ausübt, der Sequenz Nr. 8 entspricht, welche dem Fragment entspricht, das die Nucleotide 1413 bis 1461 der in Abbildung 3 dargestellten Sequenz umfasst.

18. DNA-Sequenz nach einem der Ansprüche 1 oder 2, **dadurch gekennzeichnet, dass** die Nucleotidsequenz, welche eine Funktion auf posttranskriptioneller Ebene ausübt, der Sequenz Nr. 9 entspricht, welche dem folgenden DNA-Fragment entspricht:

5'-AGCTTGAAAGGAGGGATGCCTAAAAACGAAGAACTGCA-3'
3'-ACTTTCCTCCCTACGGATTTTTGCTTCTTG-5'

19. DNA-Sequenz nach einem der Ansprüche 1 oder 2, **dadurch gekennzeichnet, dass** die Nucleotidsequenz, welche eine Funktion auf posttranskriptioneller Ebene ausübt, der Sequenz Nr. 10 entspricht, welche dem folgenden DNA-Fragment entspricht:

5'-CTTGAAAGGAGGGATGCCTAAAAACGAAGAAC-3'
3'-GAACTTTCCTCCCTACGGATTTTTGCTTCTTG-5'

20. DNA-Sequenz nach einem der Ansprüche 1 oder 2, **dadurch gekennzeichnet, dass** die Nucleotidsequenz, welche eine Funktion auf posttranskriptioneller Ebene ausübt, dem folgenden DNA-Fragment entspricht:

5'-GAAAGGAGG-3'
3'-CTTTCCTCC-5'

21. DNA-Sequenz nach einem der Ansprüche 1 bis 20, **dadurch gekennzeichnet, dass** der Promotor in der Sequenz enthalten ist, welche durch die Restriktionsschnittstellen TaqI-PacI begrenzt ist.

22. DNA-Sequenz nach Anspruch 21, **dadurch gekennzeichnet, dass** der Promotor der Sequenz Nr. 3 entspricht, welche von den Nucleotiden 907 bis 990 der Sequenz der Abbildung 3 umfasst ist oder eine Variante, die die Nucleotide 907 bis 985 umfasst oder **dadurch gekennzeichnet, dass** der Promotor in jedem Teil mindestens 10 natürlicherweise aufeinanderfolgenden oder nicht natürlicherweise aufeinanderfolgenden Nucleotiden dieser Sequenz entspricht und in der Lage ist, in die Expressionskontrolle in einer bakteriellen Wirtszelle einer codierenden Nucleotidsequenz, welche stromabwärts platziert ist, einzugreifen.

23. Nucleotidsequenz S2 der Sequenz: 
5'-CTTGAAAGGAGGGATGCCTAAAAACGAAGAAC-3'
3'-GAACTTTCCTCCCTACGGATTTTTGCTTCTTG-5'
enthaltend eine Region, welche ausreichend komplementär zum 3'-Ende einer ribosomalen bakteriellen 16S-RNA ist, vorzugsweise eines Gram+-Bakteriums, mehr bevorzugt eines Bakteriums des Typs Bacillus, um in der Lage zu sein, an ein bakterielles Ribosom oder ein Teil eines bakteriellen Ribosoms zu hybridisieren.

24. Nucleotidsequenz **dadurch gekennzeichnet, dass** sie in der Sequenz Nr. 5 enthalten ist, welche dem Fragment entspricht, das die Nucleotide 1179 bis 1556 der in Abbildung 3 dargestellten Sequenz umfasst, und dass sie eine Region enthält, welche im Wesentlichen komplementär zum 3'-Ende einer bakteriellen ribosomalen 16S-RNA ist, wobei die Sequenz in der Lage ist, auf postranskriptioneller Ebene, während der Expression der codierenden Sequenz eines Gens einzugreifen, insbesondere ohne direkt auf die Translation der zu exprimierenden codierenden Sequenz einzuwirken, wobei die Nucleotidsequenz unter der Kontrolle eines Promotors ist, welcher die codierende Sequenz kontrolliert.

25. Nucleotidsequenz nach Anspruch 24, **dadurch gekennzeichnet, dass** die Nucleotidsequenz der Sequenz Nr. 5 entspricht, welche dem Fragment entspricht, das die Nucleotide 1179 bis 1556 der in Abbildung 3 dargestellten Sequenz umfasst.

26. Nucleotidsequenz nach Anspruch 24, **dadurch gekennzeichnet, dass** die Nucleotidsequenz der Sequenz Nr. 11 entspricht, welche dem Fragment entspricht, das die Nucleotide 1413 bis 1556 der in Abbildung 3 dargestellten Sequenz umfasst.

27. Nucleotidsequenz nach Anspruch 24, **dadurch gekennzeichnet, dass** die Nucleotidsequenz der Sequenz Nr. 8 entspricht, welche dem Fragment entspricht, das die Nucleotide 1413 bis 1461 der in Abbildung 3 dargestellten Sequenz umfasst.

28. Nucleotidsequenz nach Anspruch 24, **dadurch gekennzeichnet, dass** die Nucleotidsequenz, welche eine Funktion auf posttranskriptioneller Ebene ausübt, der Sequenz Nr. 9 entspricht, welche dem folgenden DNA-Fragment entspricht:

5'-AGCTTGAAAGGAGGGATGCCTAAAAACGAAGAACTGCA-3'
3'-ACTTTCCTCCCTACGGATTTTTGCTTCTTG-5'

29. Nucleotidsequenz nach Anspruch 24, **dadurch gekennzeichnet, dass** die Nucleotidsequenz dem folgenden DNA-Fragment entspricht.

5'-GAAAGGAGG-3'
3'-CTTTCCTCC-5'

30. Rekombinante DNA-Sequenz, **dadurch gekennzeichnet, dass** sie zusammengesetzt ist aus:
(a) einer bestimmten codierenden Sequenz,
(b) einer Nucleotidsequenz zusammengesetzt aus einem Promotor und einer Sequenz, welche eine Funktion auf posttranskriptioneller Ebene ausübt gemäß einem der Ansprüche 12 bis 20, wobei die Sequenz, welche auf posttranskriptioneller Ebene eine Funktion ausübt, sich stromabwärts des Promotors und stromaufwärts der codierenden Sequenz befindet.

31. Rekombinante DNA-Sequenz, **dadurch gekennzeichnet, dass** sie umfasst:
(a) eine bestimmte codierende Sequenz unter der Kontrolle eines Promotors,
(b) eine Nucleotidsequenz, welche auf posttranskriptioneller Ebene eine Funktion ausübt gemäß einem der Ansprüche 23 bis 29, wobei die Sequenz, welche auf posttranskriptioneller Ebene eine Funktion ausübt, sich stromabwärts des Promotors und stromabwärts der codierenden Sequenz befindet.

32. Rekombinante DNA-Sequenz nach Anspruch 31, **dadurch gekennzeichnet, dass** sie zusätzlich eine Sequenz umfasst, welche eine Funktion auf posttranskriptioneller Ebene ausübt und welche sich stromaufwärts der codierenden Sequenz befindet.

33. Rekombinante DNA-Sequenz nach einem der Ansprüche 30 bis 32, **dadurch gekennzeichnet, dass** die enthaltene codierende Nucleotidsequenz eine Sequenz ist, welche für ein Enzym codiert.

34. Rekombinante DNA-Sequenz nach einem der Ansprüche 30 bis 32, **dadurch gekennzeichnet, dass** die enthaltene codierende Nucleotidsequenz eine Sequenz ist, die für ein Antigen codiert.

35. Rekombinante DNA-Sequenz nach einem der Ansprüche 30 bis 32, **dadurch gekennzeichnet, dass** die enthaltene codierende Nucleotidsequenz eine Sequenz ist, die toxisch gegenüber Insekten ist, und vorzugsweise eine Sequenz mit larventötender Aktivität ist.

36. Expressionsvektor erhalten durch Insertion einer DNA-Sequenz gebildet aus einer rekombinanten Sequenz nach einem der Ansprüche 5 bis 32, dergestalt, dass die rekombinante DNA-Sequenz in die Expressionskontrolle einer codierenden Sequenz von bestimmten Nucleotiden eingreift.

37. Expressionsvektor nach Anspruch 36, **dadurch gekennzeichnet, dass** es sich um ein Plasmid handelt, z.B. ein integratives Plasmid oder ein replikatives Plasmid.

38. Expressionsvektor nach Anspruch 37, **dadurch gekennzeichnet, dass** es sich um das Plasmid pHT7902'lacZ, hinterlegt bei der CNCM am 20. April 1993 unter der Nr. I-1301 handelt.

39. Rekombinante Wirtszelle, **dadurch gekennzeichnet, dass** sie durch eine DNA-Sequenz nach einem der Ansprüche 1 bis 35 oder durch einen Expressionsvektor nach einem der Ansprüche 36 bis 38 modifiziert ist.

40. Wirtszelle nach Anspruch 39, **dadurch gekennzeichnet, dass** es sich um ein Bacillus handelt, z. B. B. thuringiensis oder B. subtilis.

41. Wirtszelle nach Anspruch 40, **dadurch gekennzeichnet, dass** es sich um einen nicht-sporulierenden Stamm von Bacillus handelt, z.B. um einen nicht-sporulierenden Stamm von B. subtilis, vorzugsweise einen Stamm, welcher in der Lage ist, die codierende Sequenz der DNA-Sequenz nach einem der Ansprüche 14 bis 18 während der stationären Wachstumsphase zu exprimieren.

42. Wirtszelle **dadurch gekennzeichnet, dass** es sich um den Stamm 407-OA::KM^{R} (pHT305D) handelt, welcher bei der CNCM am 3. Mai 1994 unter der Nr. I-1412 hinterlegt wurde.

43. Verfahren zur Herstellung eines rekombinanten Proteins, codiert von einer bestimmten Nucleotidsequenz, wobei das Verfahren umfasst:
- die Einführung eines Vektors nach einem der Ansprüche 36 bis 38 in eine Wirtszelle,
- das Wachstum der Wirtszelle unter Bedingungen, welche die Expression der bestimmten Nucleotidsequenz erlauben, und
- das Gewinnen des rekombinanten Proteins.

44. Verwendung einer Nucleotidsequenz nach einem der Ansprüche 1 bis 22 zur Kontrolle der Expression der codierenden Sequenz eines Gens.

45. Verwendung einer Nucleotidsequenz nach einem der Ansprüche 1 bis 22 zum Erreichen eines hohen Expressionsgrades der codierenden Sequenz eines Gens.

46. Verwendung einer Nucleotidsequenz nach einem der Ansprüche 23 bis 29 zur posttranskripitionellen Regulation des Transkriptionsprodukts der codierenden Sequenz eines Gens.

47. Verwendung einer Nucleotidsequenz nach einem der Ansprüche 23 bis 29, zur Erhöhung der mRNA-Menge einer codierenden Sequenz eines Gens.

## Claims

1. A DNA sequence controlling expression of a coding sequence of a gene in a host cell, **characterized in that** it is made up of
a) a promoter; and
b) a nucleotide sequence playing a post-transcriptional role, constituted by part of a DNA fragment of about 1692 bp delimited by the HindIII and Pst1 restriction sites (H₂-P₁ fragment) shown in Figure 3A, and comprising a region complementary to the 3' end of a bacterial ribosomal 16S RNA, said sequence playing a post-transcriptional role being located downstream and under the control of said promoter.

2. A DNA sequence according to claim 1, **characterized in that** the DNA concatenation of about 1692 bp delimited by the HindIII and Pst1 restriction sites (H₂-P₁ fragment) is that obtained by partial digestion of a 6 kb BamHI fragment carrying the cryIIIA gene of Bacillus thuringiensis strain LM79, said DNA sequence also being capable of intervening in controlling expression of a downstream nucleotide coding sequence in a bacterial host cell, in particular of the Bacillus thuringiensis and/or Bacillus subtilis type.

3. A DNA sequence according to claim 1, **characterized in that** it corresponds to the HindIII - Pst1 concatenation of about 1692 bp (H₂-P₁ fragment) shown in Figure 3A.

4. A DNA sequence according to claim 1, **characterized in that** it corresponds to the following concatenation of nucleotides, designated by SEQ ID NO: 1, included between nucleotides 1 and 1692:

AAGCTTTCAG TGAAGTACGT GATTATACGG AGATGAAAAT TCGTACACTG TTAACGAGAA 60

GGAAACGCCG ACGAAAGCGT AGCATCGGAT GGCAAAGATG GAGTAACGAA TATCTCTACG 120

GTGTACTGGG GCTTTACTGA GACTAGAAAG TCCTTCCCTT GAAAAGTGCA GAGAGTTTTC 180
GATAAAAGTG TCAGCCATTT GATAAGTCTC ATTCTCATAA CCTATTGATG AAGTTTATAG 240
GGAAGCTGCT TGAGAGGGAA AACCTCACGA ACAGTTCTTA TGGGGAGAGA CTGGAAACAG 300
GTCACAATTG ATACCTCGCT AATCTTTTAA CCGACAAAGT TTTTTTAAAC CGTGGAAGTC 360
ATAATAACCT GGATATTGTG AATTTATAAA AGTTAACAAA TGGTTTATAT TAAGACAGTC 420
ATAAACCAAA GATTTTTCTT CTAAAGCTAC GATAGCAAAA ATTTCACTAG AAATTAGTTA 480
TACAAGCATT TTGTAAGAAT TATTAAAAAG ATAAATCCTG CTATTACGAG ATTAGTAGGA 540
TGATATTGTG AAAAATTTTT TATCTATTCG ATTTAAAATA TTTATGAATT TTACATAAAC 600
CTCATAAGAA AAAATACTAT CTATACTATT TTAAGAAATT TATTAGAATA AGCGGATTCA 660
AAATAGCCCT GGCCATAAAA GTACCTCAGC AGTAGAAGTT TTGACCAAAA TTAAAAAAAT 720
ACCCAATCAA GAGAATATTC TTAATTACAA TACGTTTTGC GAGGAACATA TTGATTGAAA 780
TTTAATAAAT TTAGTCCTAA AATTTAAAGA AATTTAAGTT TTTCATATTT TTATGAACTA 840
ACAAGAATAA AAATTGTGTT TATTTATTAT TCTTGTTAAA TATTTGATAA AGAGATATAT 900
TTTTGGTCGA AACGTAAGAT GAAACCTTAG ATAAAAGTGC TTTTTTTGTT GCAATTGAAG 960
AATTATTAAT GTTAAGCTTA ATTAAAGATA ATATCTTTGA ATTGTAACGC CCCTCAAAAG 1020
TAAGAACTAC AAAAAAAGAA TACGTTATAT AGAAATATGT TTGAACCTTC TTCAGATTAC 1080
AAATATATTC GGACGGACTC TACCTCAAAT GCTTATCTAA CTATAGAATG ACATACAAGC 1140
ACAACCTTGA AAATTTGAAA ATATAACTAC CAATGAACTT GTTCATGTGA ATTATCGCTG 1200
TATTTAATTT TCTCAATTCA ATATATAATA TGCCAATACA TTGTTACAAG TAGAAATTAA 1260
GACACCCTTG ATAGCCTTAC TATACCTAAC ATGATGTAGT ATTAAATGAA TATGTAAATA 1320
TATTTATGAT AAGAAGCGAC TTATTTATAA TCATTACATA TTTTTCTATT GGAATGATTA 1380
AGATTCCAAT AGAATAGTGT ATAAATTATT TATCTTGAAA GGAGGGATGC CTAAAAACGA 1440
AGAACATTAA AAACATATAT TTGCACCGTC TAATGGATTT ATGAAAAATC ATTTTATCAG 1500
TTTGAAAATT ATGTATTATG ATAAGAAAGG GAGGAAGAAA AATGAATCCG AACAATCGAA 1560
GTGAACATGA TACAATAAAA ACTACTGAAA ATAATGAGGT GCCAACTAAC CATGTTCAAT 1620
ATCCTTTAGC GGAAACTCCA AATCCAACAC TAGAAGATTT AAATTATAAA GAGTTTTTAA 1680
GAATGACTGC AG 1692

5. A DNA sequence according to claim 1 or claim 2, **characterized in that** it corresponds to a concatenation delimited by the TaqI - TaqI restriction sites.

6. A DNA sequence according to claim 1 or claim 2, **characterized in that** it corresponds to the nucleotide concatenation designated by SEQ ID NO: 2, included between nucleotides 907 and 1559 of the sequence of Figure 3.

7. A DNA sequence according to claim 1 or claim 2, **characterized in that** it comprises the TaqI-PacI fragment having the concatenation SEQ ID NO: 3 in association with the XMnI-TaqI fragment having the concatenation SEQ ID NO: 4.

8. A DNA sequence according to any one of claims 1 to 7, **characterized in that** it is involved in controlling transcription of the nucleotide coding sequence and can enhance expression of a coding sequence.

9. A DNA sequence according to claims 1 to 8, **characterized by**:
• its hybridization capacity under non stringent conditions with a nucleotide concatenation selected from concatenations SEQ ID NO: 1, SEQ ID NO: 2, SEQ ID NO: 3 and SEQ ID NO: 4; and by
• its capacity to intervene in controlling expression, in a host cell, particularly of the Bacillus type, of a downstream nucleotide coding sequence.

10. A sequence according to claim 1, **characterized in that** said nucleotide sequence playing a post-transcriptional role is the following sequence S2: 
5'-CTTGAAAGGAGGGATGCCTAAAAACGAAGAAC-3'
3'-GAACTTTCCTCCCTACGGATTTTTGCTTCTTG-5'
isolated from a bacterium, particularly from a gram+ bacterium, more particularly from a bacterium of the Bacillus type, or a sequence derived from said sequence S2, said sequence S2 being **characterized in that** it comprises a region which is essentially complementary to the 3' end of the 16S RNA of a bacterial ribosome, and **in that** it is capable of intervening at a post transcriptional level during expression of said coding sequence, especially without acting directly on translation of the coding sequence to be expressed, in particular when said sequence S2 is located between said promoter and said coding sequence of a gene, to enhance expression of said coding sequence in a bacterium, particularly a bacterium of the Bacillus type.

11. A sequence according to claim 10, **characterized in that** it comprises a first region which is sufficiently complementary to the 3' end of the 16S RNA of a bacterial ribosome, in particular a Gram+ bacterium, more particularly a bacterium of the Bacillus type, so that said first region is capable of hybridizing to a bacterial ribosome or to a portion of said ribosome, and a second region downstream of the first region, said second region comprising a sequence that is capable of having a positive additional effect on the expression of the coding sequence.

12. A DNA sequence according to claim 1, **characterized in that** the nucleotide sequence playing a post-transcriptional role has the following properties:
• it is contained in a concatenation of nucleotides hybridizing under non stringent conditions with the DNA fragment included between nucleotides 1413 and 1559 of the sequence shown in Figure 3;
• it is capable of intervening in controlling expression of the coding sequence of a gene in a host cell, in particular of a sequence encoding a polypeptide of Bacillus which is toxic to insects or of a sequence encoding a polypeptide expressed during the vegetative stage, during the stationary phase or during sporulation of Bacillus.

13. A DNA sequence according to claim 1 or claim 2, **characterized in that** the nucleotide sequence playing a post-transcriptional role corresponds to the concatenation delimited by the restriction sites XmnI-TaqI.

14. A DNA sequence according to claim 1 or claim 2, **characterized in that** the nucleotide sequence playing a post-transcriptional role corresponds to the concatenation SEQ ID NO: 4 included between nucleotides 1179 and 1559 of the sequence of Figure 3, or **in that** it corresponds to any portion of at least 10 nucleotides which may or may not be naturally consecutive of said concatenation, which is capable of intervening in controlling the expression of a downstream nucleotide coding sequence in a host cell.

15. A DNA sequence according to claim 1 or claim 2, **characterized in that** the nucleotide sequence playing a post transcriptional role corresponds to the concatenation SEQ ID NO: 5 corresponding to the fragment comprising nucleotides 1179 to 1556 of the sequence shown in Figure 3.

16. A DNA sequence according to claim 1 or claim 2, **characterized in that** the nucleotide sequence playing a post transcriptional role corresponds to the concatenation SEQ ID NO: 11 corresponding to the fragment comprising nucleotides 1413 to 1556 of the sequence shown in Figure 3.

17. A DNA sequence according to claim 1 or claim 2, **characterized in that** the nucleotide sequence playing a post transcriptional role corresponds to the concatenation SEQ ID NO: 8 corresponding to the fragment comprising nucleotides 1413 to 1461 of the sequence shown in Figure 3.

18. A DNA sequence according to claim 1 or claim 2, **characterized in that** the nucleotide sequence playing a post transcriptional role corresponds to the concatenation SEQ ID NO: 9 corresponding to the following DNA fragment:

5'-AGCTTGAAAGGAGGGATGCCTAAAAACGAAGAACTGCA-3'
3'-ACTTTCCTCCCTACGGATTTTTGCTTCTTG-5'

19. A DNA sequence according to claim 1 or claim 2, **characterized in that** the nucleotide sequence playing a post transcriptional role corresponds to the concatenation SEQ ID NO: 10 corresponding to the following DNA fragment:

5'-CTTGAAAGGAGGGATGCCTAAAAACGAAGAAC-3'
3'-GAACTTTCCTCCCTACGGATTTTTGCTTCTTG-5'

20. A DNA sequence according to claim 1 or claim 2, **characterized in that** the nucleotide sequence playing a post transcriptional role corresponds to the following DNA fragment:

5’-GAAAGGAGG-3’
3’-CTTTCCTCC-5’

21. A DNA sequence according to any one of claims 1 to 20, **characterized in that** the promoter is included in the concatenation delimited by restriction sites TaqI-PacI.

22. A DNA sequence according to claim 21, **characterized in that** the promoter corresponds to the concatenation SEQ ID NO: 3 included between nucleotides 907 and 990 of the sequence of Figure 3, or a variant comprising nucleotides 907 to 985, or **characterized in that** the promoter corresponds to any part of at least 10 nucleotides which may or may not be naturally consecutive of said concatenation, which may intervene in controlling expression of a downstream nucleotide coding sequence in a bacterial host cell.

23. A nucleotide sequence S2 with the following sequence: 
5'-CTTGAAAGGAGGGATGCCTAAAAACGAAGAAC-3'
3'-GAACTTTCCTCCCTACGGATTTTTGCTTCTTG-5'
comprising a region which is sufficiently complementary to the 3' end of the 16S RNA of a bacterial ribosome, particularly a Gram+ bacterium, more particularly a bacterium of the Bacillus type, to be capable of hybridizing to a bacterial ribosome or to a portion of a bacterial ribosome.

24. A nucleotide sequence, **characterized in that** it is comprised in the concatenation SEQ ID NO: 5 corresponding to the fragment comprising nucleotides 1179 to 1556 of the sequence shown in Figure 3, and **in that** it comprises a region which is essentially complementary to the 3' end of the 16S RNA of a bacterial ribosome, said sequence being capable of intervening at a post-transcriptional level during expression of the coding sequence of a gene, in particular without acting directly on translation of the coding sequence to be expressed, said nucleotide sequence being under the control of a promoter controlling said coding sequence.

25. A nucleotide sequence according to claim 24, **characterized in that** the nucleotide sequence corresponds to the concatenation SEQ ID NO: 5 corresponding to the fragment comprising nucleotides 1179 to 1556 of the sequence shown in Figure 3.

26. A nucleotide sequence according to claim 24, **characterized in that** the nucleotide sequence corresponds to the concatenation SEQ ID NO: 11 corresponding to the fragment comprising nucleotides 1413 to 1556 of the sequence shown in Figure 3.

27. A nucleotide sequence according to claim 24, **characterized in that** the nucleotide sequence corresponds to the concatenation SEQ ID NO: 8 corresponding to the fragment comprising nucleotides 1413 to 1461 of the sequence shown in Figure 3.

28. A nucleotide sequence according to claim 24, **characterized in that** the nucleotide sequence playing a post-transcriptional role corresponds to the concatenation SEQ ID NO: 9 corresponding to the following DNA fragment:

5'-AGCTTGAAAGGAGGGATGCCTAAAAACGAAGAACTGCA-3'
3'-ACTTTCCTCCCTACGGATTTTTGCTTCTTG-5'

29. A nucleotide sequence according to claim 24, **characterized in that** the nucleotide sequence corresponds to the following DNA fragment:

5’-GAAAGGAGG-3’

3’-CTTTCCTCC-5’

30. A recombinant DNA sequence, **characterized in that** it is constituted by:
a. a predetermined coding sequence;
b. a nucleotide sequence constituted by a promoter and a sequence playing a post-transcriptional role, according to any one of claims 12 to 20;
said sequence playing a post-transcriptional role being located downstream of said promoter and upstream of said coding sequence.

31. A recombinant DNA sequence, **characterized in that** it comprises:
a. a predetermined coding sequence, under the control of a promoter;
b. a nucleotide sequence playing a post-transcriptional role according to any one of claims 23 to 29;
said sequence playing a post-transcriptional role being located downstream of said promoter and downstream of said encoding sequence.

32. A recombinant DNA sequence according to claim 32, **characterized in that** it further comprises a sequence playing a post-transcriptional role located upstream of said coding sequence.

33. A recombinant DNA sequence according to one of claims 30 to 32, **characterized in that** the nucleotide coding sequence it contains is a sequence encoding an enzyme.

34. A recombinant DNA sequence according to one of claims 30 to 32, **characterized in that** the nucleotide coding sequence it contains is a sequence encoding an antigen.

35. A recombinant DNA sequence according to one of claims 30 to 32, **characterized in that** the nucleotide coding sequence it contains is a sequence which is toxic to insects, in particular a sequence with larvicidal activity.

36. An expression vector obtained by inserting a DNA sequence made up of a recombinant sequence according to any one of claims 5 to 32, such that said recombinant DNA sequence intervenes in controlling expression of a predetermined nucleotide coding sequence.

37. An expression vector according to claim 36, **characterized in that** it is a plasmid, for example an integrative plasmid or a replicative plasmid.

38. An expression vector according to claim 37, **characterized in that** it is plasmid pHT7902'lacZ deposited with the CNCM on 20^{th} April 1993 with accession number I-1301.

39. A recombinant host cell, **characterized in that** it is modified by a DNA sequence according to any one of claims 1 to 35 or by an expression vector according to any one of claims 36 to 38.

40. A host cell according to claim 39, **characterized in that** it is a Bacillus, for example B. thuringiensis or B. subtilis.

41. A host cell according to claim 40, **characterized in that** it is an asporogenic strain of Bacillus, for example an asporogenic strain of B. subtilis, in particular a strain capable of expressing the coding sequence of the DNA sequence according to any one of claims 14 to 18, during the stationary growth phase.

42. A host cell, **characterized in that** it is the 407 OA::Km^{R} (pHT305D) strain deposited at the CNCM on 3^{rd} May 1994 with accession number I-1412.

43. A method for producing a recombinant protein encoded by a predetermined nucleotide sequence, said method being **characterized in that** it comprises:
• introducing a vector according to any one of claims 36 to 38 into a host cell;
• growing said host cell under conditions allowing expression of said predetermined nucleotide sequence; and
• recovering the recombinant protein.

44. Use of a nucleotide sequence according to one of claims 1 to 22, to control expression of the coding sequence of a gene.

45. Use of a nucleotide sequence according to one of claims 1 to 22, to obtain a high level of expression of the coding sequence of a gene.

46. Use of a nucleotide sequence according to one of claims 23 to 29, for post-transcriptional regulation of the transcription product of the coding sequence of a gene.

47. Use of a nucleotide sequence according to any one of claims 23 to 29 to enhance the quantity of mRNA of an coding sequence of a gene.
